# EUROPEAN PATENT APPLICATION

(11) **EP 4 830 538 A1**
(43) Date of publication of application: **28.10.2026**
(21) Application number: 24907575.5
(22) Date of filing: 23.12.2024
(51) Int. Cl.: A23L 33/105

(54) **ANTI-INFLAMMATORY OR AGING-SUPPRESSING COMPOSITION CONTAINING EXTRACELLULAR VESICLES DERIVED FROM PLANT, MUSHROOM OR MICROALGA**

(30) Priority: 22.12.2023 JP 2023217413
(71) Applicant: The University of Tokyo, Bunkyo-ku, Tokyo 113-8654 (JP)
(72) Inventor: KOJIMA, Takuya, Tokyo 113-8654 (JP); AKIYAMA, Tetsu, Tokyo 113-8654 (JP); KAMOSHIDA, Yuki, Tokyo 113-8654 (JP); HAYASHI, Tomoatsu, Tokyo 113-8654 (JP); YAMAZUMI, Yusuke, Tokyo 113-8654 (JP); ODA, Takeaki, Tokyo 113-8654 (JP); SHIKANAI, Mima, Tokyo 113-8654 (JP)
(74) Representative: Weickmann & Weickmann PartmbB
(86) International application number: PCT/JP2024/045560
(87) International publication number: WO 2025/135193

(57) **Abstract**

An anti-inflammatory or aging-suppressing composition, comprising an extracellular vesicle derived from a plant of the family Apiaceae, the family Lamiaceae, the family Theaceae, the family Selaginellaceae, the family Brassicaceae, the family Asteraceae, the family Amaranthaceae, the family Amaryllidaceae, the family Fabaceae, the family Cucurbitaceae, the family Zingiberaceae, the family Polygonaceae, the family Dioscoreaceae, the family Vitaceae, the family Rosaceae, the family Solanaceae, the family Ericaceae, the family Lythraceae, or the family Rutaceae, a mushroom, or a microalga.

## Description

### [Technical Field]

The present invention relates to an anti-inflammatory or aging-suppressing composition comprising extracellular vesicles derived from a plant, a mushroom, or a microalga.

### [Background Art]

Extracellular vesicles (EVs) is a general term for vesicles enclosed by a lipid bilayer and secreted from cells in various organisms such as animals and plants, and includes exosomes derived from endosomal membranes and the like. Proteins, lipids, nucleic acids, and the like are stored inside EVs.

EVs are also known to have various physiological activities (see Patent Literatures 1 to 5).

### [Citation List]

### [Patent Literature]

[Patent Literature 1]
   JP 2018-531932 A
[Patent Literature 2]
   JP 2020-535189 A
[Patent Literature 3]
   JP 2021-193070 A
[Patent Literature 4]
   JP 2022-525131 A
[Patent Literature 5]
   JP 2022-543061 A

A problem to be solved by the present invention is to provide a novel composition capable of suppressing inflammation or aging.

### [Summary of Invention]

### [Solution to Problem]

The present inventors have conducted intensive studies in an attempt to solve the aforementioned problems and found that extracellular vesicles derived from specific plants, mushrooms, or microalgae have a potent suppressive action on inflammation or aging, which resulted in the completion of the present invention.

That is, the present invention provides the following.
[1] An anti-inflammatory or aging-suppressing composition, comprising an extracellular vesicle derived from a plant of the family Apiaceae, the family Lamiaceae, the family Theaceae, the family Selaginellaceae, the family Brassicaceae, the family Asteraceae , the family Amaranthaceae, the family Amaryllidaceae, the family Fabaceae, the family Cucurbitaceae, the family Zingiberaceae, the family Polygonaceae, the family Dioscoreaceae, the family Vitaceae, the family Rosaceae, the family Solanaceae, the family Ericaceae, the family Lythraceae, or the family Rutaceae, a mushroom, or a microalga.
[2] An anti-inflammatory or aging-suppressing composition, comprising an extracellular vesicle derived from parsley, ashitaba, Japanese honeywort, Violet Harmony, carrot, edible carrot leaf, fruit carrot, coriander, dill, fennel, rosemary, spearmint, lemon balm, perilla, marjoram, sweet basil, thyme, oregano, peppermint, sage, tea plant, camellia, Temarikatahiba, Brussels sprouts, cauliflore, romanesco, arugula, horseradish, Japanese mustard spinach, wasabi, cauliflower, watermelon radish, perennial wall-rocket, bok choy, broccoli, potherb mustard, radish, Japanese radish, watercress, turnip, tatsoi, artichoke, Okinawan spinach, garland chrysanthemum, Korean lettuce, burdock, endive, Hakata Jerusalem artichoke, romaine lettuce, Italian chicory, Mexican tea, amaranthus, spinach, Swiss chard, beet, saltwort, Chinese chive, leek, onion, shallot, Romano bean, green soybean, winged bean, snow pea, snap pea, pea shoot, watermelon, zucchini, sponge gourd, ginger, Japanese ginger, rhubarb, Japanese yam, muscat, mulberry, bell pepper, chili pepper, potato, green pepper, blueberry, pomegranate, kaffir lime, kumquat, white mushroom, shiitake mushroom, brown beech mushroom, or Euglena.
[3] The composition of [1] or [2], wherein the composition suppresses expression of an inflammatory cytokine or an aging marker.
[4] The composition of [3], wherein the inflammatory cytokine or aging marker is IL-4, IL-6, IL-13, IL-33, IL-1β, CXCL1, CXCL2, TNF-α, p15, or p16.
[5] The composition of any of [1] to [4], for a food, a cosmetic, or a pharmaceutical product.

The present invention includes the following embodiments.
[A1] A method for suppressing inflammation or aging, comprising using an extracellular vesicle derived from a plant of the family Apiaceae, the family Lamiaceae, the family Theaceae, the family Selaginellaceae, the family Brassicaceae, the family Asteraceae, the family Amaranthaceae, the family Amaryllidaceae, the family Fabaceae, the family Cucurbitaceae, the family Zingiberaceae, the family Polygonaceae, the family Dioscoreaceae, the family Vitaceae, the family Rosaceae, the family Solanaceae, the family Ericaceae, the family Lythraceae, or the family Rutaceae, a mushroom, or a microalga.
[A2] A method for suppressing inflammation or aging, comprising using an extracellular vesicle derived from parsley, ashitaba, Japanese honeywort, Violet Harmony, carrot, edible carrot leaf, fruit carrot, coriander, dill, fennel, rosemary, spearmint, lemon balm, perilla, marjoram, sweet basil, thyme, oregano, peppermint, sage, tea plant, camellia, Temarikatahiba, Brussels sprouts, cauliflore, romanesco, arugula, horseradish, Japanese mustard spinach, wasabi, cauliflower, watermelon radish, perennial wall-rocket, bok choy, broccoli, potherb mustard, radish, Japanese radish, watercress, turnip, tatsoi, artichoke, Okinawan spinach, garland chrysanthemum, Korean lettuce, burdock, endive, Hakata Jerusalem artichoke, romaine lettuce, Italian chicory, Mexican tea, amaranthus, spinach, Swiss chard, beet, saltwort, Chinese chive, leek, onion, shallot, Romano bean, green soybean, winged bean, snow pea, snap pea, pea shoot, watermelon, zucchini, sponge gourd, ginger, Japanese ginger, rhubarb, Japanese yam, muscat, mulberry, bell pepper, chili pepper, potato, green pepper, blueberry, pomegranate, kaffir lime, kumquat, white mushroom, shiitake mushroom, brown beech mushroom, or Euglena.

[B1] Use of an extracellular vesicle derived from a plant of the family Apiaceae, the family Lamiaceae, the family Theaceae, the family Selaginellaceae, the family Brassicaceae, the family Asteraceae, the family Amaranthaceae, the family Amaryllidaceae, the family Fabaceae, the family Cucurbitaceae, the family Zingiberaceae, the family Polygonaceae, the family Dioscoreaceae, the family Vitaceae, the family Rosaceae, the family Solanaceae, the family Ericaceae, the family Lythraceae, or the family Rutaceae, a mushroom, or a microalga, for suppressing inflammation or aging.
[B2] Use of an extracellular vesicle derived from parsley, ashitaba, Japanese honeywort, Violet Harmony, carrot, edible carrot leaf, fruit carrot, coriander, dill, fennel, rosemary, spearmint, lemon balm, perilla, marjoram, sweet basil, thyme, oregano, peppermint, sage, tea plant, camellia, Temarikatahiba, Brussels sprouts, cauliflore, romanesco, arugula, horseradish, Japanese mustard spinach, wasabi, cauliflower, watermelon radish, perennial wall-rocket, bok choy, broccoli, potherb mustard, radish, Japanese radish, watercress, turnip, tatsoi, artichoke, Okinawan spinach, garland chrysanthemum, Korean lettuce, burdock, endive, Hakata Jerusalem artichoke, romaine lettuce, Italian chicory, Mexican tea, amaranthus, spinach, Swiss chard, beet, saltwort, Chinese chive, leek, onion, shallot, Romano bean, green soybean, winged bean, snow pea, snap pea, pea shoot, watermelon, zucchini, sponge gourd, ginger, Japanese ginger, rhubarb, Japanese yam, muscat, mulberry, bell pepper, chili pepper, potato, green pepper, blueberry, pomegranate, kaffir lime, kumquat, white mushroom, shiitake mushroom, brown beech mushroom, or Euglena, for suppressing inflammation or aging.

[C1] Use of an extracellular vesicle derived from a plant of the family Apiaceae, the family Lamiaceae, the family Theaceae, the family Selaginellaceae, the family Brassicaceae, the family Asteraceae, the family Amaranthaceae, the family Amaryllidaceae, the family Fabaceae, the family Cucurbitaceae, the family Zingiberaceae, the family Polygonaceae, the family Dioscoreaceae, the family Vitaceae, the family Rosaceae, the family Solanaceae, the family Ericaceae, the family Lythraceae, or the family Rutaceae, a mushroom, or a microalga, for the production of a composition suppressing inflammation or aging.
[C2] Use of an extracellular vesicle derived from parsley, ashitaba, Japanese honeywort, Violet Harmony, carrot, edible carrot leaf, fruit carrot, coriander, dill, fennel, rosemary, spearmint, lemon balm, perilla, marjoram, sweet basil, thyme, oregano, peppermint, sage, tea plant, camellia, Temarikatahiba, Brussels sprouts, cauliflore, romanesco, arugula, horseradish, Japanese mustard spinach, wasabi, cauliflower, watermelon radish, perennial wall-rocket, bok choy, broccoli, potherb mustard, radish, Japanese radish, watercress, turnip, tatsoi, artichoke, Okinawan spinach, garland chrysanthemum, Korean lettuce, burdock, endive, Hakata Jerusalem artichoke, romaine lettuce, Italian chicory, Mexican tea, amaranthus, spinach, Swiss chard, beet, saltwort, Chinese chive, leek, onion, shallot, Romano bean, green soybean, winged bean, snow pea, snap pea, pea shoot, watermelon, zucchini, sponge gourd, ginger, Japanese ginger, rhubarb, Japanese yam, muscat, mulberry, bell pepper, chili pepper, potato, green pepper, blueberry, pomegranate, kaffir lime, kumquat, white mushroom, shiitake mushroom, brown beech mushroom, or Euglena, for the production of a composition suppressing inflammation or aging.

[D1] An extracellular vesicle derived from a plant of the family Apiaceae, the family Lamiaceae, the family Theaceae, the family Selaginellaceae, the family Brassicaceae, the family Asteraceae, the family Amaranthaceae, the family Amaryllidaceae, the family Fabaceae, the family Cucurbitaceae, the family Zingiberaceae, the family Polygonaceae, the family Dioscoreaceae, the family Vitaceae, the family Rosaceae, the family Solanaceae, the family Ericaceae, the family Lythraceae, or the family Rutaceae, a mushroom, or a microalga, for use in suppressing inflammation or aging.
[D2] An extracellular vesicle derived from parsley, ashitaba, Japanese honeywort, Violet Harmony, carrot, edible carrot leaf, fruit carrot, coriander, dill, fennel, rosemary, spearmint, lemon balm, perilla, marjoram, sweet basil, thyme, oregano, peppermint, sage, tea plant, camellia, Temarikatahiba, Brussels sprouts, cauliflore, romanesco, arugula, horseradish, Japanese mustard spinach, wasabi, cauliflower, watermelon radish, perennial wall-rocket, bok choy, broccoli, potherb mustard, radish, Japanese radish, watercress, turnip, tatsoi, artichoke, Okinawan spinach, garland chrysanthemum, Korean lettuce, burdock, endive, Hakata Jerusalem artichoke, romaine lettuce, Italian chicory, Mexican tea, amaranthus, spinach, Swiss chard, beet, saltwort, Chinese chive, leek, onion, shallot, Romano bean, green soybean, winged bean, snow pea, snap pea, pea shoot, watermelon, zucchini, sponge gourd, ginger, Japanese ginger, rhubarb, Japanese yam, muscat, mulberry, bell pepper, chili pepper, potato, green pepper, blueberry, pomegranate, kaffir lime, kumquat, white mushroom, shiitake mushroom, brown beech mushroom, or Euglena, for use in suppressing inflammation or aging.

[E1] An anti-inflammatory or aging-suppressing agent comprising an extracellular vesicle derived from a plant of the family Apiaceae, the family Lamiaceae, the family Theaceae, the family Selaginellaceae, the family Brassicaceae, the family Asteraceae, the family Amaranthaceae, the family Amaryllidaceae, the family Fabaceae, the family Cucurbitaceae, the family Zingiberaceae, the family Polygonaceae, the family Dioscoreaceae, the family Vitaceae, the family Rosaceae, the family Solanaceae, the family Ericaceae, the family Lythraceae, or the family Rutaceae, a mushroom, or a microalga.
[E2] An anti-inflammatory or aging-suppressing agent comprising an extracellular vesicle derived from parsley, ashitaba, Japanese honeywort, Violet Harmony, carrot, edible carrot leaf, fruit carrot, coriander, dill, fennel, rosemary, spearmint, lemon balm, perilla, marjoram, sweet basil, thyme, oregano, peppermint, sage, tea plant, camellia, Temarikatahiba, Brussels sprouts, cauliflore, romanesco, arugula, horseradish, Japanese mustard spinach, wasabi, cauliflower, watermelon radish, perennial wall-rocket, bok choy, broccoli, potherb mustard, radish, Japanese radish, watercress, turnip, tatsoi, artichoke, Okinawan spinach, garland chrysanthemum, Korean lettuce, burdock, endive, Hakata Jerusalem artichoke, romaine lettuce, Italian chicory, Mexican tea, amaranthus, spinach, Swiss chard, beet, saltwort, Chinese chive, leek, onion, shallot, Romano bean, green soybean, winged bean, snow pea, snap pea, pea shoot, watermelon, zucchini, sponge gourd, ginger, Japanese ginger, rhubarb, Japanese yam, muscat, mulberry, bell pepper, chili pepper, potato, green pepper, blueberry, pomegranate, kaffir lime, kumquat, white mushroom, shiitake mushroom, brown beech mushroom, or Euglena.

In each embodiment of [A1] to [E2],
the expression of an inflammatory cytokine or an aging marker may be suppressed,
the inflammatory cytokine or aging marker may be IL-4, IL-6, IL-13, IL-33, IL-1β, CXCL1, CXCL2, TNF-α, p15, or p16, and
it may be for foods, cosmetics, or pharmaceutical products.

The present invention includes the following embodiments.
[6] An anti-inflammatory or aging-suppressing composition, comprising an extracellular vesicle derived from parsley or Euglena.
[7] The composition of [6], wherein an anti-inflammatory or aging-suppressing action is not attenuated when the composition is subjected to a thermal history.
[8] The composition of [7], wherein the thermal history is heat treatment performed at 40°C or higher.

In each embodiment of [6] to [8], the composition may be thermally stable, may exhibit an anti-inflammatory action and/or an aging-suppressing action equivalent to those of an unheated composition, and may suppress the expression of an aging marker and/or a cytokine to the same extent as an unheated composition.

### [Advantageous Effects of Invention]

According to the present invention, it becomes possible to provide a novel composition comprising an extracellular vesicle capable of suppressing inflammation or aging.

### [Brief Description of Drawings]

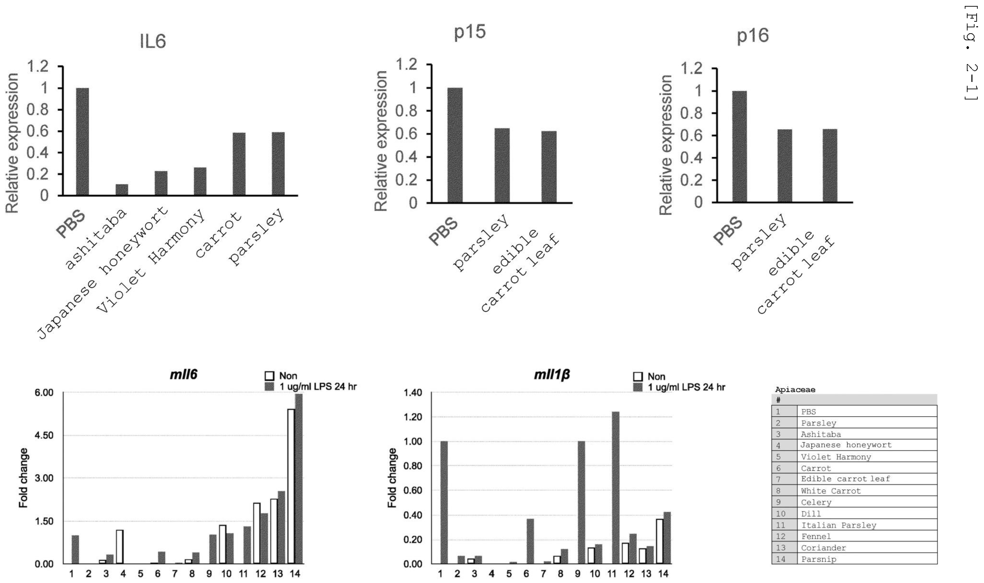

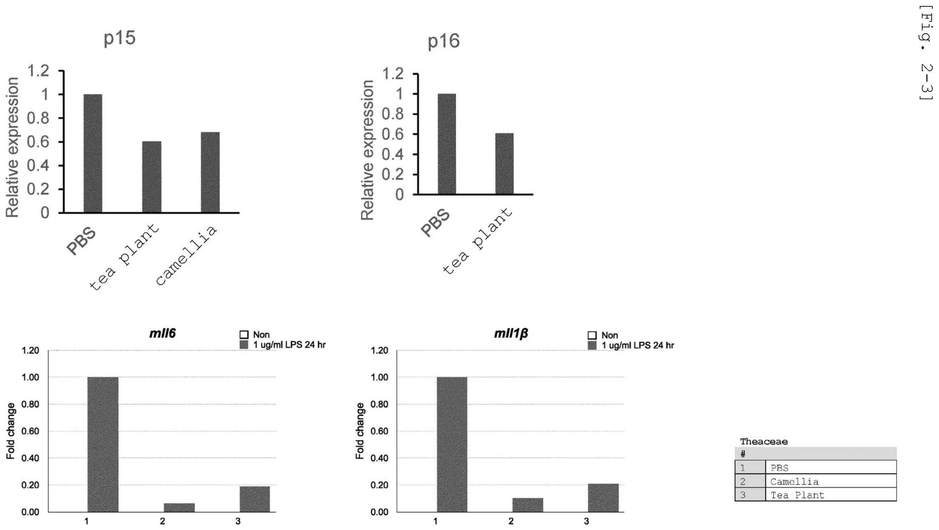

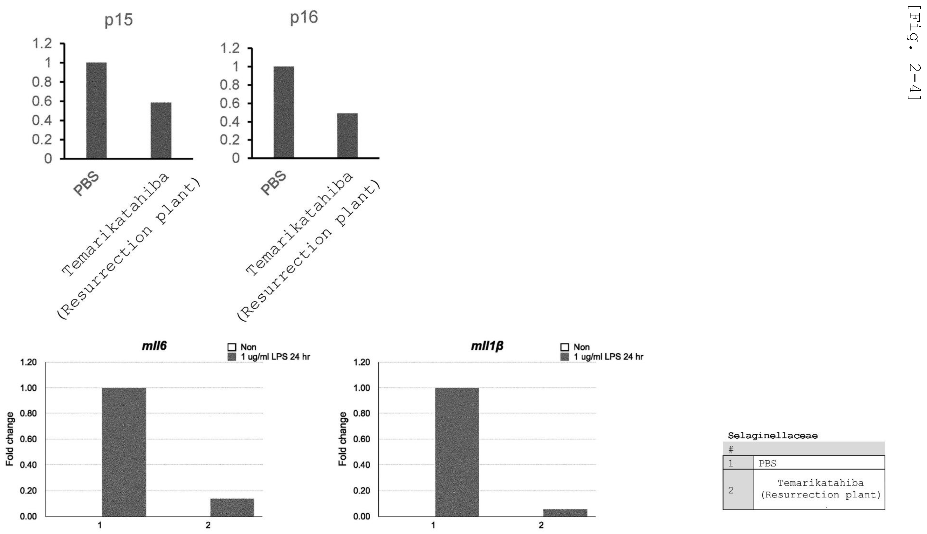

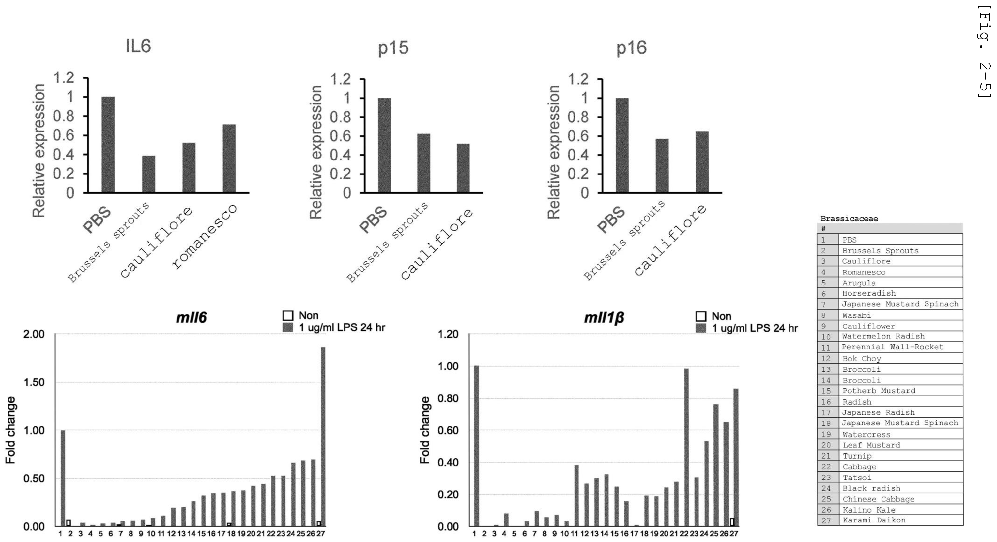

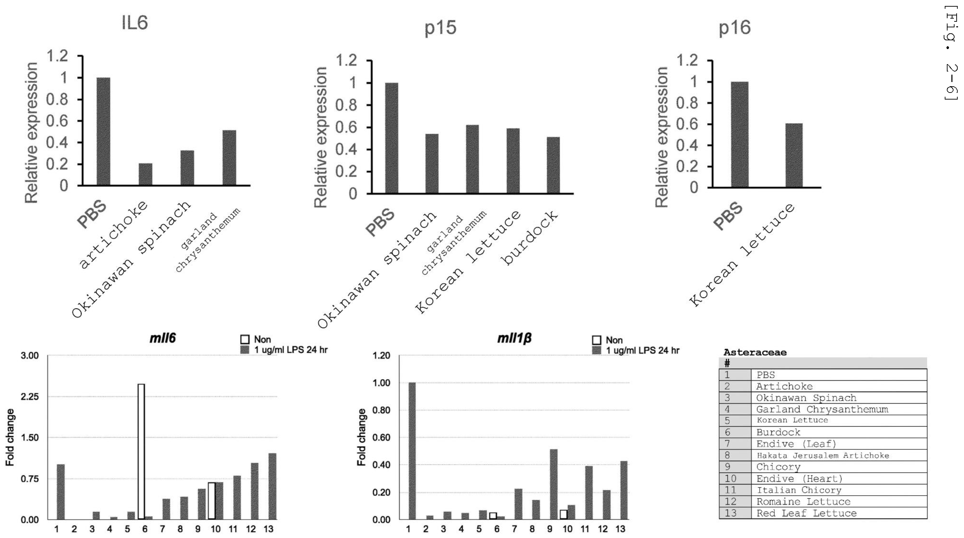

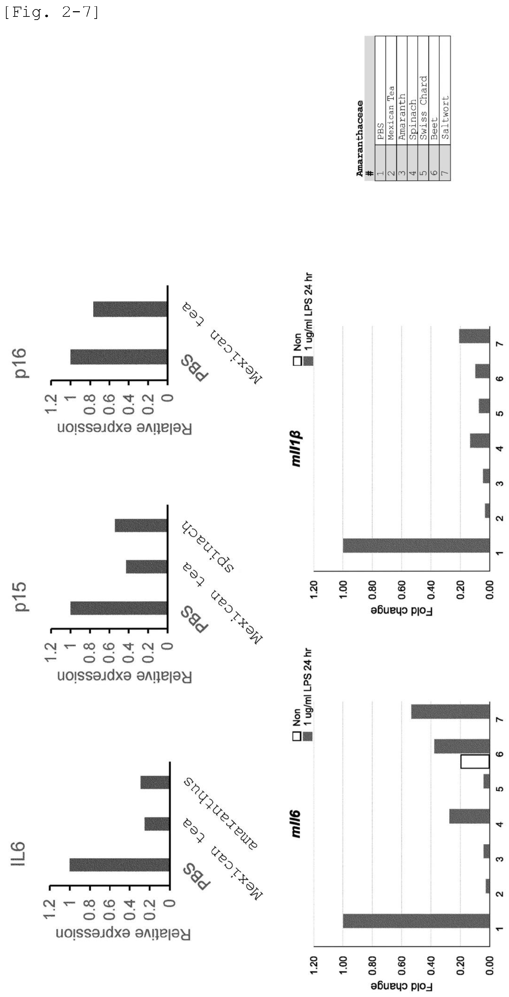

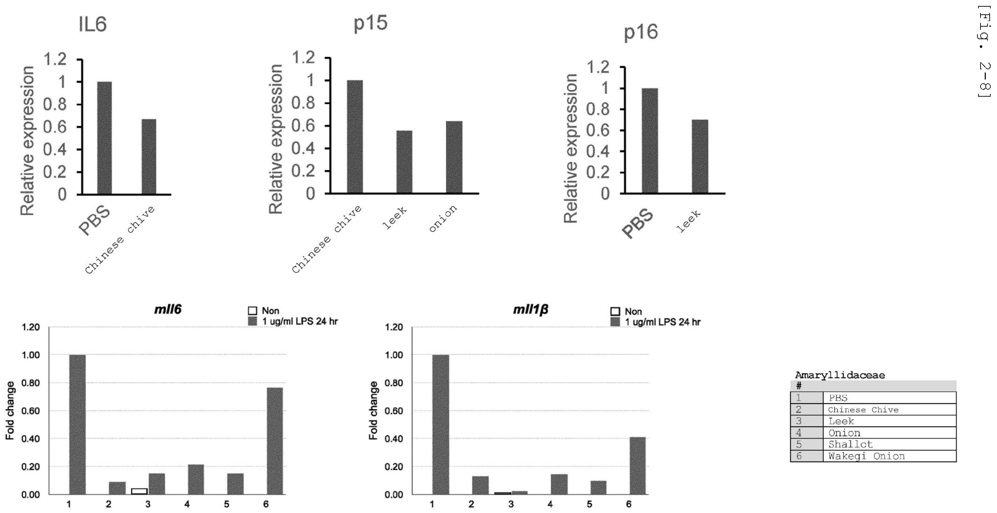

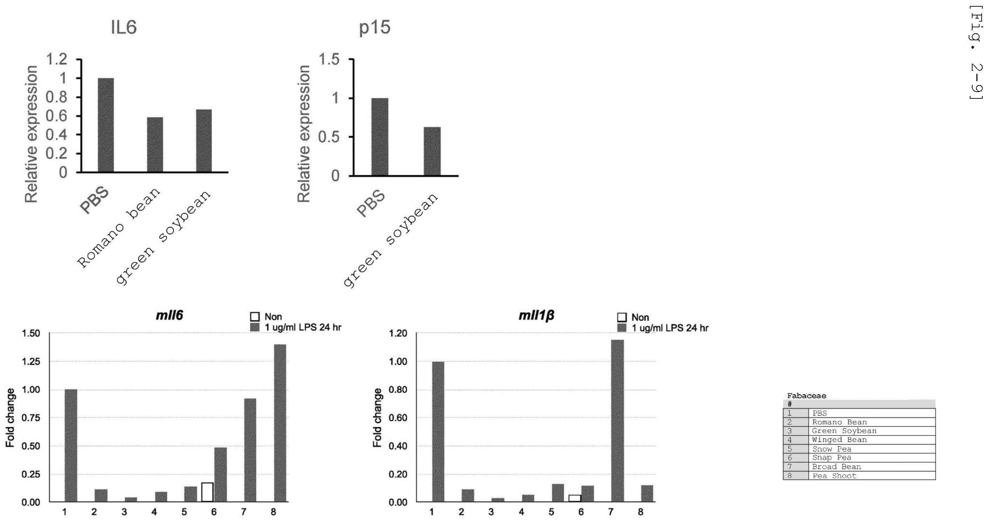

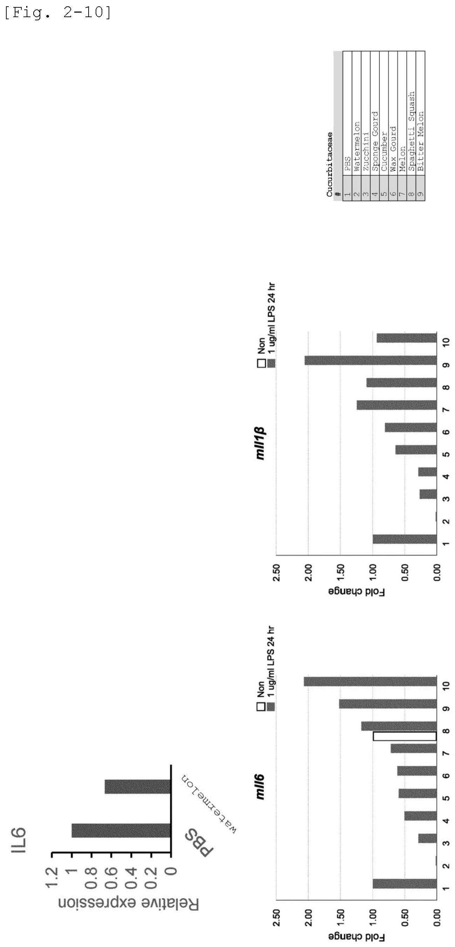

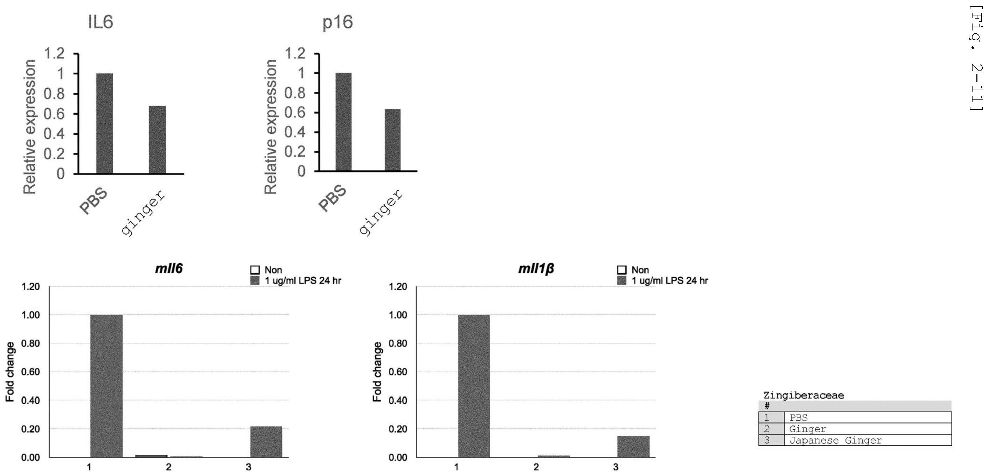

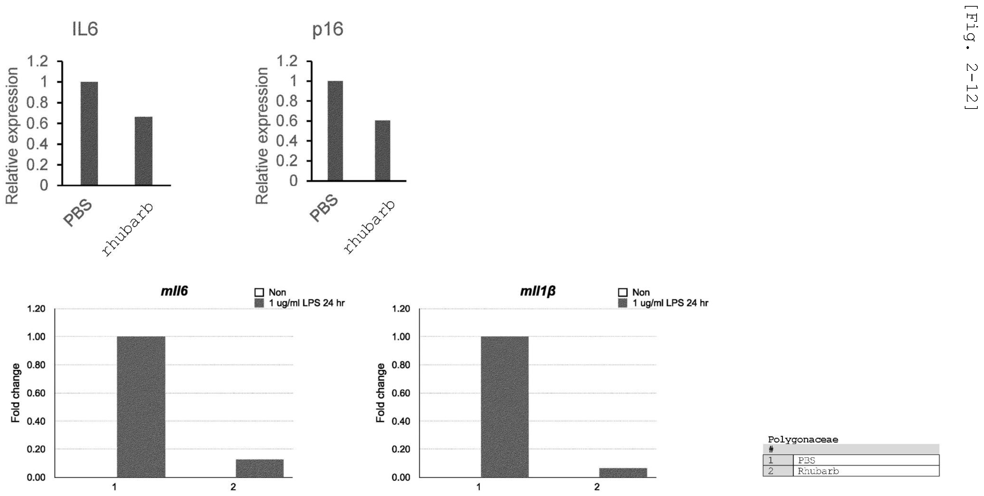

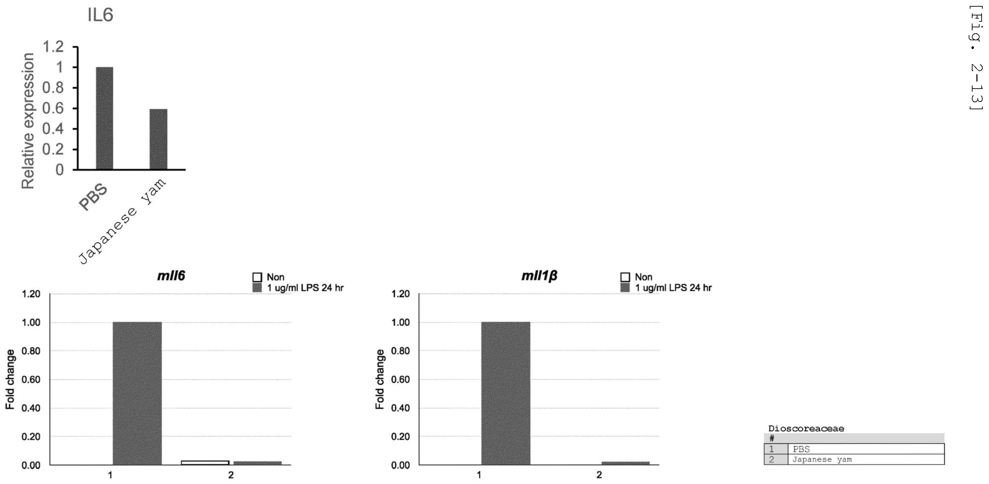

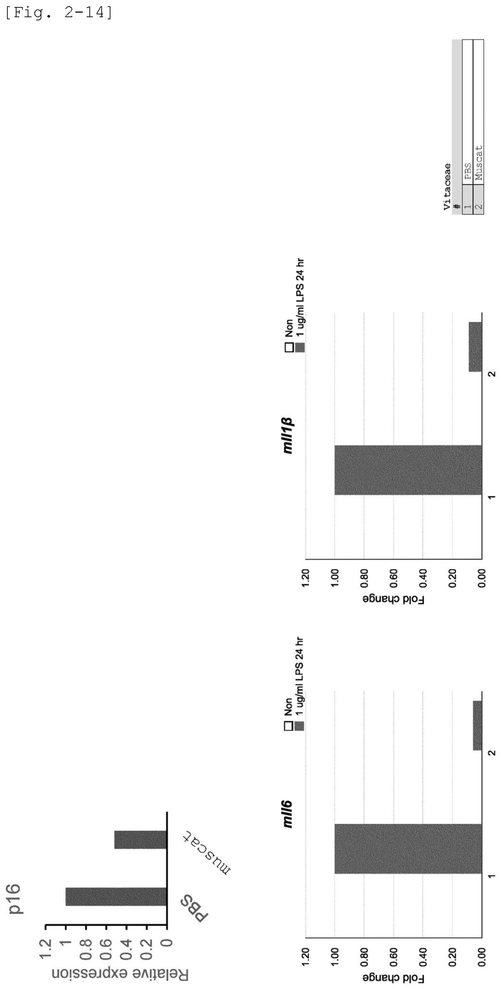

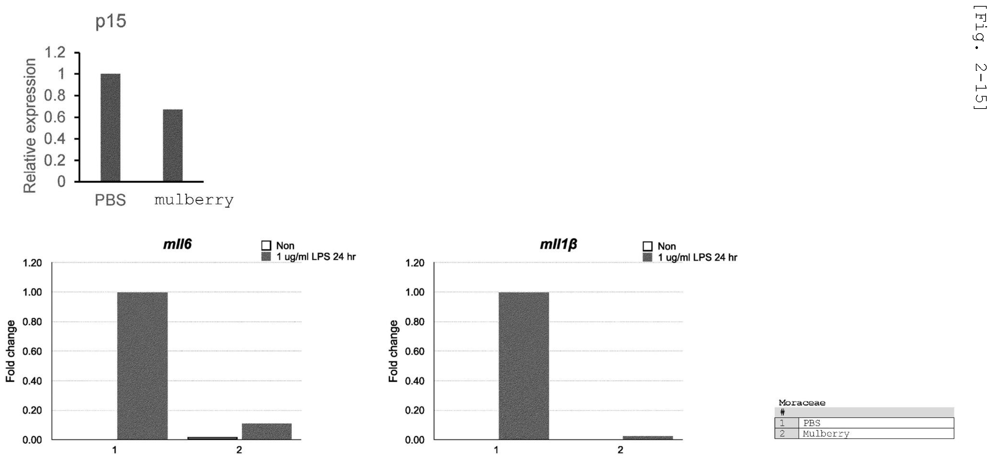

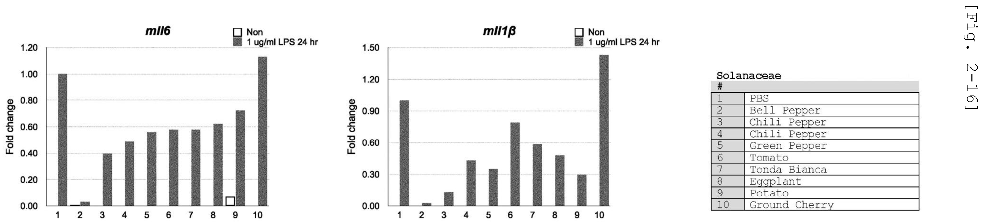

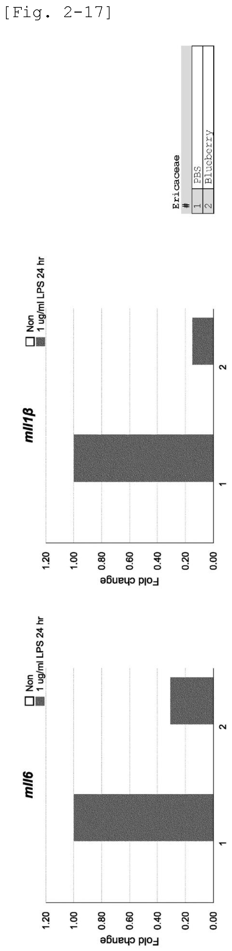

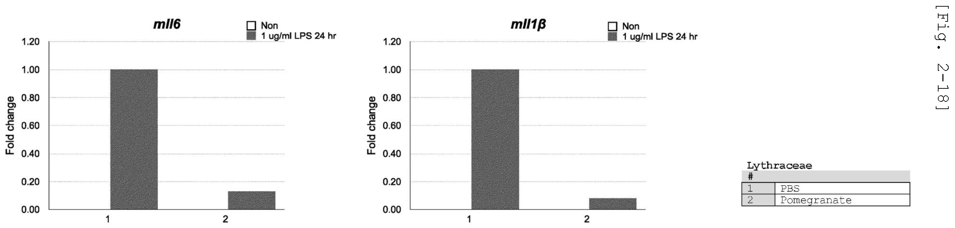

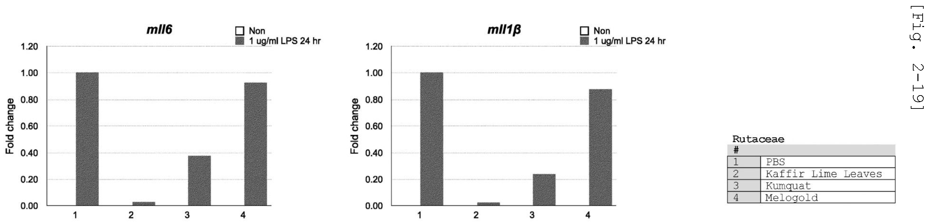

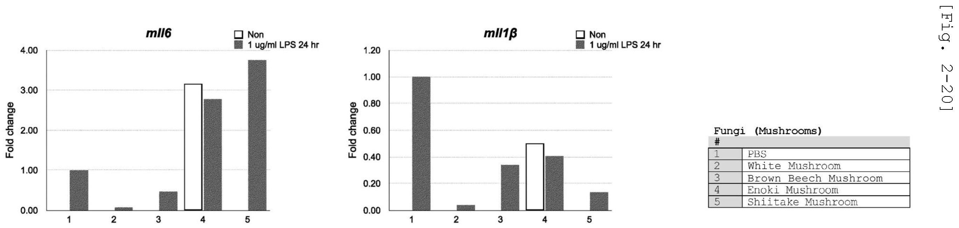

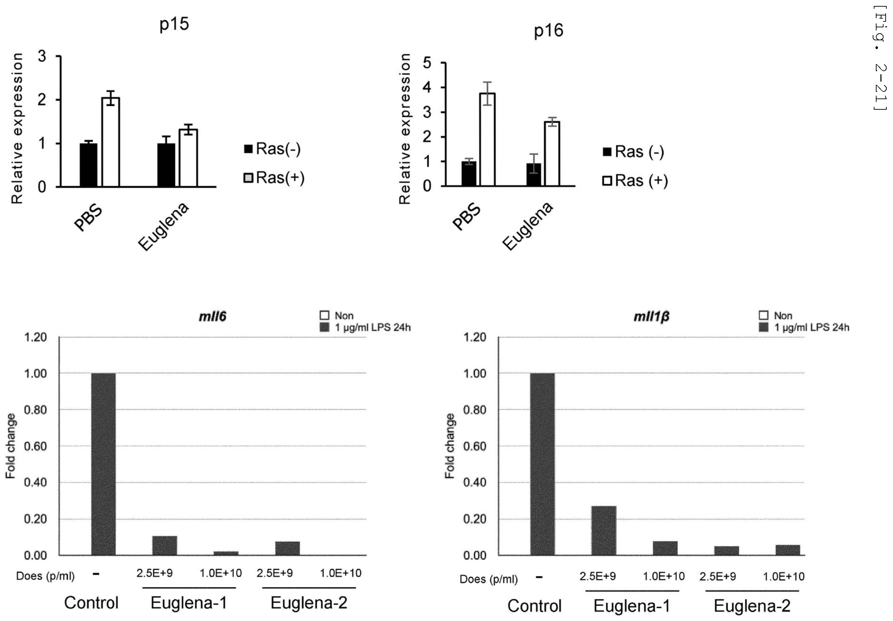

[Fig. 1]
   In preparation of EVs derived from parsley and ginger, fractions obtained by performing sucrose density gradient ultracentrifugation treatment after ultracentrifugation treatment are shown. The arrow indicates the Band 2 fraction.
[Fig. 2]
   EV fraction solutions derived from the plants, mushrooms, or microalgae listed in Table 1 were allowed to act on human IMR90 cells in which cellular senescence was induced by overexpressing active Ras. The expression levels of cellular senescence markers (IL-6, p15, and p16) were measured by qRT-PCR and the results are shown. Also, results of expression levels of inflammatory cytokines (IL-6 or IL-1β) when allowed to act on mouse macrophage cells (Raw-Blue cells) in which inflammation was induced by applying LPS stimulation are shown. In Figs. 2-1 to 2-14, 2-19 and 2-21, the upper panel shows results of the senescence induction experiment by overexpression of active Ras, and the lower panel shows results of the inflammation induction experiment by LPS stimulation.
[Fig. 3-1]
   The results of inflammatory cytokine (IL-6 or IL-1β) expression are shown for mouse macrophage cells (Raw-Blue cells) that were sensitized with an EVs fraction solution or EVs sample derived from winged bean obtained by sucrose density gradient ultracentrifugation or ultrafiltration, or sensitized with PBS, followed by LPS stimulation.
[Fig. 3-2]
   The results of inflammatory cytokine (IL-6 or IL-1β) expression are shown for mouse macrophage cells (Raw-Blue cells) that were sensitized with an EVs sample derived from parsley or Yanaka ginger obtained by ultrafiltration, or sensitized by PBS, followed by LPS stimulation.
[Fig. 4-1A]
   The results of nanoparticle tracking analysis of an EVs fraction solution derived from winged bean obtained by sucrose density gradient ultracentrifugation.
[Fig. 4-1B]
   The results of nanoparticle tracking analysis are shown for an EVs sample derived from winged bean obtained by ultrafiltration.
[Fig. 4-2A]
   The results of nanoparticle tracking analysis are shown for an EVs fraction solution derived from parsley obtained by sucrose density gradient ultracentrifugation.
[Fig. 4-2B]
   The results of nanoparticle tracking analysis are shown for an EVs sample derived from parsley obtained by ultrafiltration.
[Fig. 4-3A]
   The results of nanoparticle tracking analysis are shown for an EVs fraction solution derived from Yanaka ginger obtained by sucrose density gradient ultracentrifugation.
[Fig. 4-3B]
   The results of nanoparticle tracking analysis are shown for an EVs sample derived from Yanaka ginger obtained by ultrafiltration.
[Fig. 4-4]
   The results of nanoparticle tracking analysis are shown for an EVs fraction solution derived from Euglena obtained by sucrose density gradient ultracentrifugation.
[Fig. 5-1]
   The results of analyzing the facial skin condition (wrinkles, spots, pores, skin texture) after oral intake of parsley EVs by the skin diagnostic imaging system VISIA are shown.
[Fig. 5-2]
   The melanin accumulated in the deep layers of human skin after oral intake of parsley EVs was analyzed by the skin diagnostic imaging system VISIA.
[Fig. 6A]
   The results of inflammatory cytokine (IL-6) expression are shown for mouse macrophage cells (Raw-Blue cells) that were sensitized with non-heat-treated or heat-treated EVs samples derived from parsley obtained by ultrafiltration, followed by LPS stimulation.
[Fig. 6B]
   The results of inflammatory cytokine (IL-6) expression are shown for mouse macrophage cells (Raw-Blue cells) that were sensitized with non-heat-treated or heat-treated EVs fraction solution derived from Euglena obtained by ultracentrifugation, followed by LPS stimulation.
[Fig. 7A]
   Body weight changes are shown for mice in which colitis was induced by the administration of 3% DSS in drinking water, following one week of oral administration of parsley-derived EVs (PcELNs) at a dose of 1.0E+11 particles/day. PBS was used as a control.
[Fig. 7B]
   Phenotypes of mice after DSS intake are shown (Category 0: no phenotype, 1: diarrhea/bloody stool, 2: bleeding +, 3: bleeding ++, 4: bleeding +++, 5: anal bleeding).
[Fig. 7C]
   Lengths of the colon (from immediately below the cecum to the anus) in the PBS group and PcELNs group at the time of DSS intake are shown (Day 0: N=6, Day 3: N=3, Day 5: N=3, Day 7: N=6.).
[Fig. 7D]
   The colon from the cecum to the anus was divided into 3 parts, the side near the cecum was set as anterior, and the side near the anus was set as posterior, and measurement results by RT-qPCR are shown (N=3).
[Fig. 7E]
   Immunostaining images of HE, Alcian blue and (β-CATENIN/ZO-1) at DSS day 0 and day 7 (scale bar: 100 µm) are shown.
[Fig. 8A]
   The results of measuring thickness of auricle of mice with inflammation induced by TNCB stimulation, after EVs derived from Brussels sprouts, EVs derived from parsley, or PBS were permeated into the ears of the mice.
[Fig. 8B]
   The results of the alteration in the inflammatory cytokine expression in the ear measured by RT-PCR following the permeation treatment of a solution containing EVs after inducing inflammation, are shown.
[Fig. 9]
   The grip strength of each mouse was measured using Grip Strength Test after 13 weeks of high-fat diet and concurrent oral administration of EVs derived from parsley or Brussels sprouts. The average grip strength is shown.
[Fig. 10]
   The average muscle endurance of each mouse was measured using 4-limb Hanging Test after 13 weeks of high-fat diet and concurrent oral administration of EVs derived from parsley or Brussels sprouts. Hanging time (the duration the mouse remained suspended before falling from a wire net) and Hanging score (value obtained by multiplying Hanging time by body weight of each mouse) are shown.
[Fig. 11]
   The average weight of soleus muscle of the hind limbs for each mouse, which was fed a high-fat diet and concurrently administered EVs derived from parsley or Brussels sprouts for 13 weeks, is shown.

### [Description of Embodiments]

An embodiment for carrying out the present invention (hereinafter referred to as "the present embodiment") is described in detail in the following. The present invention is not limited to the following present embodiment.

The anti-inflammatory or aging-suppressing composition of the present embodiment contains an extracellular vesicle derived from a plant of the family Apiaceae, the family Lamiaceae, the family Theaceae, the family Selaginellaceae, the family Brassicaceae, the family Asteraceae, the family Amaranthaceae, the family Amaryllidaceae, the family Fabaceae, the family Cucurbitaceae, the family Zingiberaceae, the family Polygonaceae, the family Dioscoreaceae, the family Vitaceae, the family Rosaceae, the family Solanaceae, the family Ericaceae, the family Lythraceae, or the family Rutaceae, a mushroom, or a microalga.

The extracellular vesicles contained in the anti-inflammatory or aging-suppressing composition may contain extracellular vesicles derived from two or more different plants from the same family, and may contain extracellular vesicles derived from two or more different mushrooms or microalgae. Also, the extracellular vesicles may contain extracellular vesicles derived from one or more different plants selected from the same family, and extracellular vesicles derived from one or more plants selected from a family different from the aforementioned family. They may contain extracellular vesicles derived from plants of two or more different families. They may contain extracellular vesicles derived from plants and mushrooms, may contain extracellular vesicles derived from plants and microalgae, may contain extracellular vesicles derived from mushrooms and microalgae, and may contain extracellular vesicles derived from plants, mushrooms, and microalgae.

The plant may be a plant cultivated in an open field, or may be a plant cultivated hydroponically. The mushroom may be a natural mushroom, and may be a mushroom inoculated on an artificial medium or a felled wood. The microalgae may be cultured ones.

The extracellular vesicles of the present embodiment may be extracellular vesicles extracted by a conventionally known method using a plant, mushroom, or microalga as a raw material. Also, the extracellular vesicles derived from a plant, mushroom, or microalga may be those in which extracellular vesicles existing in the plant, mushroom, or microalga have been taken out by a conventionally known method, and since a plant, mushroom, or microalga is used as a raw material, they may contain a solvent or reagent used for extraction, and components derived from the plant, mushroom, or microalga.

The plant of the family Apiaceae is not particularly limited, and may be, for example, parsley, ashitaba, Japanese honeywort, Violet Harmony, carrot, edible carrot leaf, fruit carrot, coriander, dill, or fennel.

The plant of the family Lamiaceae is not particularly limited, and may be, for example, rosemary, spearmint, lemon balm, perilla, marjoram, sweet basil, thyme, oregano, peppermint, or sage. Perilla may be red perilla.

The plant of the family Theaceae is not particularly limited, and may be, for example, tea plant or camellia.

The plant of the family Selaginellaceae is not particularly limited, and may be, for example, Temarikatahiba. Temarikatahiba is also called resurrection plant or Rose of Jericho.

The plant of the family Brassicaceae is not particularly limited, and may be, for example, Brussels sprouts, cauliflore, romanesco, arugula, horseradish, Japanese mustard spinach, wasabi, cauliflower, watermelon radish, perennial wall-rocket, bok choy, broccoli, potherb mustard, radish, Japanese radish, watercress, turnip, or tatsoi.

The plant of the family Asteraceae is not particularly limited, and may be, for example, artichoke, Okinawan spinach, garland chrysanthemum, Korean lettuce, burdock, endive, Hakata Jerusalem artichoke, romaine lettuce, or Italian chicory.

The plant of the family Amaranthaceae is not particularly limited, and may be, for example, Mexican tea, amaranthus, spinach, Swiss chard, beet, or saltwort.

The plant of the family Amaryllidaceae is not particularly limited, and may be, for example, Chinese chive, leek, onion, or shallot.

The plant of the family Fabaceae is not particularly limited, and may be, for example, Romano bean, green soybean, winged bean, snow pea, snap pea, or pea shoot.

The plant of the family Cucurbitaceae is not particularly limited, and may be, for example, watermelon, zucchini, or sponge gourd.

The plant of the family Zingiberaceae is not particularly limited, and may be, for example, ginger or Japanese ginger.

The plant of the family Polygonaceae is not particularly limited, and may be, for example, rhubarb.

The plant of the family Dioscoreaceae is not particularly limited, and may be, for example, Japanese yam.

The plant of the family Vitaceae is not particularly limited, and may be, for example, muscat.

The plant of the family Rosaceae is not particularly limited, and may be, for example, mulberry.

The plant of the family Solanaceae is not particularly limited, and may be, for example, bell pepper, chili pepper, potato, or green pepper.

The plant of the family Ericaceae is not particularly limited, and may be, for example, blueberry.

The plant of the family Lythraceae is not particularly limited, and may be, for example, pomegranate.

The plant of the family Rutaceae is not particularly limited, and may be, for example, kaffir lime or kumquat.

The mushroom is not particularly limited, and may be, for example, white mushroom, shiitake mushroom, or brown beech mushroom. The mushroom is also called a fungal plant.

The microalga is not particularly limited, and may be, for example, Euglena.

In the present embodiment, an anti-inflammatory or aging-suppressing composition containing an extracellular vesicle derived from parsley, ashitaba, Japanese honeywort, Violet Harmony, carrot, edible carrot leaf, fruit carrot, coriander, dill, fennel, rosemary, spearmint, lemon balm, perilla, marjoram, sweet basil, thyme, oregano, peppermint, sage, tea plant, camellia, Temarikatahiba, Brussels sprouts, cauliflore, romanesco, arugula, horseradish, Japanese mustard spinach, wasabi, cauliflower, watermelon radish, perennial wall-rocket, bok choy, broccoli, potherb mustard, radish, Japanese radish, watercress, turnip, tatsoi, artichoke, Okinawan spinach, garland chrysanthemum, Korean lettuce, burdock, endive, Hakata Jerusalem artichoke, romaine lettuce, Italian chicory, Mexican tea, amaranthus, spinach, Swiss chard, beet, saltwort, Chinese chive, leek, onion, shallot, Romano bean, green soybean, winged bean, snow pea, snap pea, pea shoot, watermelon, zucchini, sponge gourd, ginger, Japanese ginger, rhubarb, Japanese yam, muscat, mulberry, bell pepper, chili pepper, potato, green pepper, blueberry, pomegranate, kaffir lime, kumquat, white mushroom, shiitake mushroom, brown beech mushroom, or Euglena is also provided.

The extracellular vesicle used in this embodiment may be an extracellular vesicle derived from parsley, Euglena, ashitaba, Japanese honeywort, Violet Harmony, edible carrot leaf, rosemary, spearmint, lemon balm, perilla, marjoram, sweet basil, thyme, oregano, peppermint, tea plant, camellia, Temarikatahiba, Brussels sprouts, cauliflore, romanesco, arugula, horseradish, Japanese mustard spinach, wasabi, cauliflower, watermelon radish, perennial wall-rocket, artichoke, Okinawan spinach, garland chrysanthemum, Korean lettuce, burdock, Mexican tea, amaranthus, Swiss chard, Chinese chive, leek, onion, shallot, Romano bean, green soybean, winged bean, snow pea, watermelon, ginger, rhubarb, Japanese yam, muscat, mulberry, bell pepper, pomegranate, kaffir lime, or mushroom. The extracellular vesicles used in the present embodiment may be any combination from the extracellular vesicles listed above.

The anti-inflammatory or aging-suppressing composition of the present embodiment has an anti-inflammatory or aging-suppressing action by containing the extracellular vesicle in the present embodiment as an active ingredient. It may have an anti-inflammatory or aging-suppressing action by containing, as an active ingredient, an extracellular vesicle derived from parsley, Euglena, ashitaba, Japanese honeywort, Violet Harmony, edible carrot leaf, rosemary, spearmint, lemon balm, perilla, marjoram, sweet basil, thyme, oregano, peppermint, tea plant, camellia, Temarikatahiba, Brussels sprouts, cauliflore, romanesco, arugula, horseradish, Japanese mustard spinach, wasabi, cauliflower, watermelon radish, perennial wall-rocket, artichoke, Okinawan spinach, garland chrysanthemum, Korean lettuce, burdock, Mexican tea, amaranthus, Swiss chard, Chinese chive, leek, onion, shallot, Romano bean, green soybean, winged bean, snow pea, watermelon, Japanese ginger, rhubarb, Japanese yam, muscat, mulberry, bell pepper, pomegranate, kaffir lime, or mushroom. It may have an anti-inflammatory or aging-suppressing action by containing, as an active ingredient, any combination from the extracellular vesicles listed above.

The concentration of the extracellular vesicle in the anti-inflammatory or aging-suppressing composition may be any content as long as it can exert its function as an active ingredient, and may be, for example, 1.0x10⁵, 1.0x10⁶, 1.0x10⁷, 1.0x10⁸, 1.0x10⁹, or 1.0x10¹⁰ particles/ml (or particles/g) or more.

In the present embodiment, "extracellular vesicles (EVs)" is a general term for vesicles enclosed by a lipid bilayer and secreted from cells in various organisms such as animals and plants, and is a concept including exosomes derived from endosomal membranes and the like. Also, in the present specification, extracellular vesicles are also indicated as EVs, and EVs have the same meaning as extracellular vesicles.

The extracellular vesicles derived from a plant, mushroom, or microalga in the present embodiment can be obtained by a method known to a person skilled in the art, and are not particularly limited. They can be obtained using, for example, ultracentrifugation, density gradient ultracentrifugation, ultrafiltration, isolation by a chromatography method, isolation using beads or antibodies, a polymer precipitation method, a microfluidic separation technique, or the like.

Specific examples of density gradient ultracentrifugation and ultrafiltration are shown below.

### Density gradient ultracentrifugation

A plant or mushroom is made into juice using a household juicer or the like. This juice is subjected to centrifugation at 1,000 to 10,000 g for 10 to 60 min, and larger debris is removed. Then, the obtained supernatant is ultracentrifuged at 120,000 to 150,000 g for 60 min to overnight to obtain a precipitate. After this precipitate is suspended in a buffer solution such as PBS, it is overlaid on a sucrose density gradient solution, and further ultracentrifuged at 120,000 to 150,000 g for 60 min to overnight. After ultracentrifugation, the target extracellular vesicles can be obtained by collecting Band 1 or Band 2 fraction accumulated at the 8-30% or 30-45% interface. Where appropriate, after the collected fraction is mixed with a buffer solution such as PBS, a precipitate obtained by further ultracentrifugation may be used as a final extracellular vesicle sample.

Microalgae are subjected to centrifugation at 1,000 to 3,000 g for 10 to 60 min, and a precipitate is suspended in a buffer solution such as PBS. The suspension is, as it is, or after freezing and thawing, crushed with an ultrasonic crusher, and thereafter, centrifugation is performed at 1,000 to 10,000 g for 10 to 60 min, and larger debris is removed. Then, the obtained supernatant is ultracentrifuged at 120,000 to 150,000 g for 60 min to overnight, to obtain a precipitate. After this precipitate is suspended in a buffer solution such as PBS, it may be used as an extracellular vesicle sample, or it is overlaid on a sucrose density gradient solution, and further ultracentrifuged at 120,000 to 150,000 g for 60 min to overnight. After ultracentrifugation, the target extracellular vesicles can be obtained by collecting Band 1 or Band 2 fraction accumulated at the 8-30% or 30-45% interface. Where appropriate, after the collected fraction is mixed with a buffer solution such as PBS, a precipitate obtained by further ultracentrifugation may be used as a final extracellular vesicle sample.

### Ultrafiltration

First, a plant, mushroom, or microalga is made into juice using a household juicer or the like. This juice is subjected to centrifugation at 1,000 to 10,000 g for 10 to 60 min, and larger debris is removed. The obtained supernatant is subjected to ultrafiltration treatment using an ultrafiltration membrane according to the size of the target extracellular vesicles, whereby the target extracellular vesicles are obtained. Here, filtration treatment may be performed using two or more types of ultrafiltration membranes.

The extracellular vesicles derived from parsley or Euglena that are contained in the composition in the present embodiment are extracellular vesicles obtained using parsley or Euglena as a raw material, and may be obtained by density gradient ultracentrifugation, or may be obtained by ultrafiltration. Also, they may be extracellular vesicles obtained by combining the both methods.

The extracellular vesicles derived from parsley or Euglena may be extracellular vesicles obtained by the aforementioned treatment.

The extracellular vesicles derived from parsley or Euglena may be extracellular vesicles having heat resistance, as shown in the Examples, and may have a property that an anti-inflammatory or aging-suppressing action is not reduced when they are subjected to a thermal history. The thermal history may be 35°C or higher, 40°C or higher, 50°C or higher, 55°C or higher, 60°C or higher, 65°C or higher, 70°C or higher, 75°C or higher, 80°C or higher, 85°C or higher, 90°C or higher, or 95°C or higher, and may be 140°C or lower, 130°C or lower, 120°C or lower, 110°C or lower, or 100°C or lower. That is, the extracellular vesicles derived from parsley or Euglena in the present embodiment may be stable to heat, and may be stable, for example, to heat of 60°C, 65°C, 70°C, 75°C, 80°C, 85°C, 90°C, or 95°C or higher. The temperature referred to here is generally an external temperature, but may be an internal temperature.

In the present embodiment, since the extracellular vesicles derived from parsley or Euglena have heat resistance, they have resistance to sterilization treatment and the like, and have a property that the anti-inflammatory action or the aging-suppressing action possessed by the extracellular vesicles is not reduced.

That the action is not reduced means, for example, that measurement data under temperature conditions appropriate for measuring each action (for example, room temperature, the temperature described in the Examples, or the temperature described in the protocol for action measurement) are not reduced under predetermined heating conditions, and for example, may be within a range of an increase/decrease value of ±20%, or may be within a range of an increase/decrease value within 10%, relative to the original measurement data. In addition, the heated extracellular vesicles derived from parsley or Euglena may be stable to heat, may show an anti-inflammatory action and/or aging-suppressing action equivalent to those of extracellular vesicles derived from parsley or Euglena that have not been heated, and may suppress expression of senescence markers and cytokines equivalently to extracellular vesicles derived from parsley or Euglena that have not been heated. Alternatively, for EVs derived from parsley or Euglena, the value obtained by dividing the expression level of an aging marker or cytokine in cells treated with heat-treated EVs by that in cells treated with non-heat-treated EVs may be smaller than that for EVs not derived from parsley or Euglena. When the anti-inflammatory or aging-suppressing composition in the present embodiment contains extracellular vesicles derived from parsley or Euglena that are stable to heat, it becomes possible to conveniently perform heat sterilization without losing activity, for example, before commercialization.

The size of extracellular vesicles obtained from a plant, mushroom, or microalga can be analyzed by a method known to a person skilled in the art, and for example, the particle diameter, particle size distribution, and concentration of extracellular vesicles can be examined by nanoparticle tracking analysis (NTA) and the like.

Extracellular vesicles obtained using density gradient ultracentrifugation have, for example, a particle diameter of 10 to 500 nm, and the concentration of extracellular vesicles may become large at a particle diameter of 50 to 450 nm. In addition, extracellular vesicles obtained using ultrafiltration have, for example, a particle diameter of 10 to 300 nm, and the concentration of extracellular vesicles may become large at a particle diameter of 50 to 250 nm. Extracellular vesicles having a more limited particle diameter range may be obtained using ultrafiltration than using density gradient ultracentrifugation.

In the present embodiment, "anti-inflammatory" means, for example, an action of suppressing inflammation, an action of preventing further inflammation, an action of preventing inflammation, and the like.

In the "anti-inflammatory action" exhibited by the composition in the present embodiment, for example, by suppressing expression in cells of cytokines inducing inflammation (IL-4, IL-6, IL-13, IL-33, IL-1β, CXCL1, CXCL2, TNF-α, and the like), release of cytokines is suppressed, whereby an anti-inflammatory action may be exhibited.

Also, for example, by the anti-inflammatory action, swelling, pain, fever and the like due to inflammation can be relieved, and inflammation not accompanied by symptoms such as swelling can also be suppressed.

The composition in the present embodiment can be applied for treatment or prevention of various inflammations. The inflammation may be, for example, acute inflammation or chronic inflammation, and also may be, for example, inflammation due to an allergic disease or autoimmune disease. In addition, the composition in the present embodiment can be applied for treatment or prevention of inflammatory diseases, for example, dermatitis, bronchitis, arthritis, gastritis, hepatitis, nephritis, encephalitis, systemic lupus erythematosus, ulcerative colitis, Crohn's disease, and dry eye, and diseases related to inflammation such as diabetes, decreased renal function, decreased liver function, cardiovascular disease, arteriosclerosis, arteriosclerosis, cerebral infarction, neurodegenerative disease, Alzheimer's disease, Parkinson's disease, narcolepsy, or cancer, and further, decreased immunity, decline in muscle strength/muscle endurance, sarcopenia (which may be sarcopenia due to aging), frailty, osteoporosis, arthropathy such as knee osteoarthritis, spinal deformity, tendon degeneration, weight loss, skin spots, wrinkles, presbyopia, and the like. Furthermore, the composition in the present embodiment can also be applied for treatment or prevention of novel coronavirus infection and its sequelae, and other infectious diseases.

The composition in the present embodiment can exhibit an anti-inflammatory action by being administered directly or indirectly to an affected part.

Here, the composition in the present embodiment can also suppress aging related to inflammation by suppressing inflammation. That is, the composition in the present embodiment has an aging-suppressing action, and in the present embodiment, when it has an anti-inflammatory action, it can also be said to have an aging-suppressing action. Aging suppression means suppressing symptoms associated with aging or senescence.

The relationship between inflammation and aging is described below.

Cellular senescence, which plays an important role in aging of an individual (Baker et al., Nature 479:232-236 2011.), is considered to be a chronic inflammatory state. Also, since senescent cells show increased expression of cellular senescence markers such as p16, p15 and p21 in the cells, and secrete inflammatory cytokines (for example, IL-6, IL-8 and the like) and the like, it is suggested that inflammation is related to onset of aging-related diseases (Birch et al., Genes Dev. 34:1565-1576 2020., Sharpless et al., Nat. Rev. Cancer 15:397-408 2015., and van Deursen, Nature 509:439-446 2014.).

In the present embodiment, "aging suppression" means an action of suppressing aging, an action of preventing aging, and the like. "Aging suppression" can be used interchangeably with "anti-aging". By the "aging-suppressing action" exhibited by the composition in the present embodiment, for example, symptoms related to cellular senescence can be suppressed. For example, symptoms related to aging of epithelial cells, bone cells, cartilage cells, nerve cells, muscle cells, adipocytes, hepatocytes, lymphocytes, and the like, for example, wrinkles, neurodegenerative disease, Alzheimer's disease, Parkinson's disease, narcolepsy, decline in muscle strength/muscle endurance, sarcopenia, frailty, osteoporosis, arthropathy such as knee osteoarthritis, spinal deformity, tendon degeneration, weight loss, diabetes, decreased immunity, decreased renal function, decreased liver function, cardiovascular disease, arteriosclerosis, cerebral infarction, and cancer can be suppressed. Furthermore, symptoms due to novel coronavirus infection and its sequelae, and other infectious diseases can also be suppressed. Also, as a result, effects such as prolongation of life span can also be obtained.

In the "aging-suppressing action," for example, by suppressing expression in cells of senescence markers (p15, p16, IL-6) or cytokines (IL-4, IL-6, IL-13, IL-33, IL-1β, CXCL1, CXCL2, TNF-α, and the like), release of cytokines may be suppressed, whereby an aging-suppressing action may be exhibited. That is, by the composition in the present embodiment, expression in cells of senescence markers (IL-6, p15, p16 and the like) or cytokines (IL-4, IL-6, IL-13, IL-33, IL-1β, CXCL1, CXCL2, TNF-α and the like) is suppressed, and release of cytokines is suppressed.

The composition in the present embodiment can exhibit an anti-inflammatory action or an aging-suppressing action by being administered directly or indirectly to an affected part.

For example, when the expression level of cytokines can be suppressed to 90%, 80%, 70%, 60%, 50%, 40%, 30%, 20%, or 10% or less compared with the case where the composition in the present embodiment is not added or administered, or the like, the presence of an anti-inflammatory or aging-suppressing action can be determined. Preferably, when the expression level of cytokines can be suppressed to 70% or less in the case of a senescence induction experiment (p15, p16, IL-6), and to 40% or less in the case of an inflammation induction experiment (IL-6, IL-1β), compared with the case where the composition in the present embodiment is not added or administered and the like, the presence of an aging-suppressing action or an anti-inflammatory action can be determined.

The anti-inflammatory composition of the present embodiment may have both an anti-inflammatory action and an aging-suppressing action. In addition, the aging-suppressing composition in the present embodiment may have both an anti-inflammatory action and an aging-suppressing action. That is, the anti-inflammatory composition may be an aging-suppressing composition, and the aging-suppressing composition may be an anti-inflammatory composition.

The anti-inflammatory composition or the aging-suppressing composition of the present embodiment may have a skin-improving effect, and for example, by administering the anti-inflammatory composition or the aging-suppressing composition, whitening or skin turnover may be promoted, and pores, spots (senile lentigines, melasma, Post-Inflammatory Hyperpigmentation), latent spots (invisible dark spots), redness, dullness, texture, firmness, sagging and the like may be improved. Those effects can be evaluated by a method known to a person skilled in the art, and can be evaluated, for example, using an image diagnostic apparatus and the like.

The composition in the present embodiment may be for food use, cosmetic use, or pharmaceutical use. That is, the present embodiment also provides an anti-inflammatory or aging-suppressing composition for food use, cosmetic use, or pharmaceutical use.

The composition can have, for example, a form suitable for the embodiment of use such as food use, cosmetic use, or pharmaceutical use, and is not particularly limited. For example, a form suitable for the embodiment of use such as a solid form, liquid form, granular form, particulate form, powder form, capsule form, cream form, paste form, or jelly form. In addition, for example, when making a capsule form, it may be coated with one or more layers of membrane as necessary. In order to make these forms, conventionally known additives can be used for the production. While they are not particularly limited, excipients, binders, diluents, disintegrants, buffers, thickeners, stabilizers, emulsifiers, dispersants, suspending agents, preservatives and the like known to a person skilled in the art may be used as necessary.

The composition for food use is not particularly limited, and examples thereof include foods and drinks, health foods, functional foods, foods for specified health uses, foods with nutrient function claims, health supplement foods, supplements, and the like.

The composition for cosmetic use is not particularly limited, and examples thereof include scalp cosmetics, skin cosmetics, finishing cosmetics, perfumes, nail cosmetics, bath cosmetics, and the like.

Examples of dosage forms of the composition for pharmaceutical use include oral agents such as tablet, capsule, granule, fine granule, powder, liquid, syrup, chewable, and troche, ointments, gels, creams, injections, sublingual agents, inhalants, suppositories, coating agents, patches, eye drops/nasal drops, and the like. The above dosage forms may be applied to a composition for food use or a composition for cosmetic use.

When the composition is provided, indications such as "suppress inflammation", "for those concerned about aging", "prevention of muscle decrease", "suppression of decline in muscle strength", "increase of muscle", "enhancement of muscle strength", "improvement of muscle endurance", and "promotion of skin turnover" may be attached.

In the composition, preferably, any amount of extracellular vesicles can be contained as long as an anti-inflammatory or aging-suppressing action can be exhibited. As long as the action is not impaired, other active ingredients may also be contained.

The embodiment of the anti-inflammatory or aging-suppressing composition in the present embodiment also applies to other embodiments described below.

In other embodiment, a method for suppressing inflammation or aging, comprising using an extracellular vesicle derived from a plant of the family Apiaceae, the family Lamiaceae, the family Theaceae, the family Selaginellaceae, the family Brassicaceae, the family Asteraceae, the family Amaranthaceae, the family Amaryllidaceae, the family Fabaceae, the family Cucurbitaceae, the family Zingiberaceae, the family Polygonaceae, the family Dioscoreaceae, the family Vitaceae, the family Rosaceae, the family Solanaceae, the family Ericaceae, the family Lythraceae, or the family Rutaceae, a mushroom, or a microalga is provided. Also, in other embodiment, a method for suppressing inflammation or aging, comprising using an extracellular vesicle derived from parsley, ashitaba, Japanese honeywort, Violet Harmony, carrot, edible carrot leaf, fruit carrot, coriander, dill, fennel, rosemary, spearmint, lemon balm, perilla, marjoram, sweet basil, thyme, oregano, peppermint, sage, tea plant, camellia, Temarikatahiba, Brussels sprouts, cauliflore, romanesco, arugula, horseradish, Japanese mustard spinach, wasabi, cauliflower, watermelon radish, perennial wall-rocket, bok choy, broccoli, potherb mustard, radish, Japanese radish, watercress, turnip, tatsoi, artichoke, Okinawan spinach, garland chrysanthemum, Korean lettuce, burdock, endive, Hakata Jerusalem artichoke, romaine lettuce, Italian chicory, Mexican tea, amaranthus, spinach, Swiss chard, beet, saltwort, Chinese chive, leek, onion, shallot, Romano bean, green soybean, winged bean, snow pea, snap pea, pea shoot, watermelon, zucchini, sponge gourd, ginger, Japanese ginger, rhubarb, Japanese yam, muscat, mulberry, bell pepper, chili pepper, potato, green pepper, blueberry, pomegranate, kaffir lime, kumquat, white mushroom, shiitake mushroom, brown beech mushroom, or Euglena is also provided.

The method for suppressing inflammation or aging is performed, for example, in order to suppress inflammation or aging outside or inside the human body, and also, by carrying it out when inflammation or aging is desired to be suppressed, or when a disease caused by inflammation or aging is treated, it can be performed to suppress inflammation or aging outside or inside the human body.

In other embodiment, an anti-inflammatory agent or an aging-suppressing agent containing an extracellular vesicle derived from a plant of the family Apiaceae, the family Lamiaceae, the family Theaceae, the family Selaginellaceae, the family Brassicaceae, the family Asteraceae, the family Amaranthaceae, the family Amaryllidaceae, the family Fabaceae, the family Cucurbitaceae, the family Zingiberaceae, the family Polygonaceae, the family Dioscoreaceae, the family Vitaceae, the family Rosaceae, the family Solanaceae, the family Ericaceae, the family Lythraceae, or the family Rutaceae, a mushroom, or a microalga is provided.

In other embodiment, an anti-inflammatory agent or an aging-suppressing agent containing an extracellular vesicle derived from parsley, ashitaba, Japanese honeywort, Violet Harmony, carrot, edible carrot leaf, fruit carrot, coriander, dill, fennel, rosemary, spearmint, lemon balm, perilla, marjoram, sweet basil, thyme, oregano, peppermint, sage, tea plant, camellia, Temarikatahiba, Brussels sprouts, cauliflore, romanesco, arugula, horseradish, Japanese mustard spinach, wasabi, cauliflower, watermelon radish, perennial wall-rocket, bok choy, broccoli, potherb mustard, radish, Japanese radish, watercress, turnip, tatsoi, artichoke, Okinawan spinach, garland chrysanthemum, Korean lettuce, burdock, endive, Hakata Jerusalem artichoke, romaine lettuce, Italian chicory, Mexican tea, amaranthus, spinach, Swiss chard, beet, saltwort, Chinese chive, leek, onion, shallot, Romano bean, green soybean, winged bean, snow pea, snap pea, pea shoot, watermelon, zucchini, sponge gourd, ginger, Japanese ginger, rhubarb, Japanese yam, muscat, mulberry, bell pepper, chili pepper, potato, green pepper, blueberry, pomegranate, kaffir lime, kumquat, white mushroom, shiitake mushroom, brown beech mushroom, or Euglena is also provided.

The anti-inflammatory agent or the aging-suppressing agent is not particularly limited, and may be, for example, a dosage form such as oral agents such as tablet, capsule, granule, fine granule, powder, liquid, syrup, chewable, and troche, ointments, gels, creams, injections, sublingual agents, inhalants, and suppositories. In order to make these dosage forms, conventionally known additives can be used for the production. While they are not particularly limited, excipients, binders, diluents, disintegrants, buffers, thickeners, stabilizers, emulsifiers, dispersants, suspending agents, preservatives and the like known in the industry may be used as necessary. Also, the anti-inflammatory agent or the aging-suppressing agent may be blended into foods, cosmetics, pharmaceutical products, and the like and provided.

The composition containing the anti-inflammatory agent may be an anti-inflammatory composition, and the composition containing the aging-suppressing agent may be an aging-suppressing composition. The anti-inflammatory or aging-suppressing composition containing the anti-inflammatory agent or the aging-suppressing agent may be for edible use, cosmetic use, or pharmaceutical use.

The anti-inflammatory or aging-suppressing composition, or the anti-inflammatory agent or the aging-suppressing agent in the present embodiment is administered to animals in general including humans. The dose and administration frequency may be appropriately set according to various conditions such as the administration route, and the age, body weight, symptoms and the like of the target subject. The target subject is preferably a mammal, more preferably a human, monkey, cat, pig, horse, cow, mouse, rat, guinea pig, dog, and rabbit, and further preferably a human.

The method for suppressing inflammation or aging may include a step of administering, to a subject, an anti-inflammatory or aging-suppressing composition, or an anti-inflammatory agent or an aging-suppressing agent.

In the present specification, the method includes a therapeutic method and a non-therapeutic method, and the use includes therapeutic use and non-therapeutic use.

### [Example]

The present invention is explained more specifically in the following by referring to specific examples. The present invention is not limited by the examples.

### <Example 1>

### (1) Evaluation of anti-aging and anti-inflammatory action of EVs in human IMR90 cells and mouse macrophage cells

### (1-1) Culture of human normal fibroblast IMR90 cells

Human normal fibroblast IMR90 cells were cultured in an incubator under conditions of 37°C, 3% O₂, 5% CO₂ using E-MEM medium (WAKO) supplemented with 10% FBS, MEM NEAA (gibco), and Sodium Pyruvate (gibco), and were subcultured at a ratio of 5x10⁵ cells/100 mm dish.

### (1-2) Establishment of IMR90 cells having oncogenic Ras inducibility

Using polyethyleneimine, plasmid pLNCX2 ER:ras (addgene) was introduced into retrovirus packaging cells Plat-A, and cultured for 1 week, whereby retrovirus containing ER:ras gene was produced in the culture supernatant of Plat-A. Thereafter, hexadimethrine bromide (Merck) was added to the culture supernatant containing this retrovirus, and the ER:ras gene was introduced into IMR90 cells by adding the supernatant to the medium of the IMR90 cells. Furthermore, by adding G418 (gibco) to the medium of the IMR90 cells, only IMR90 cells with the ER:ras gene introduced thereinto were selected, and an IMR90 cell line in which cellular senescence can be induced by the Ras gene was established.

### (1-3) Culture of mouse macrophage cells

Mouse macrophage cells (Raw-Blue cells, manufactured by InvivoGen) were cultured in an incubator under conditions of 37°C, 5% CO₂ using Dulbecco's Modified Eagle Medium (manufactured by Thermo Fisher) supplemented with 10% FBS, 100 µg/ml Normocin (manufactured by InvivoGen), and Pen-Strep (100 U/ml), and were subcultured at a ratio of 4.7x10⁶ cells/100 mm dish.

### (1-4) Preparation of EVs using sucrose density gradient ultracentrifugation

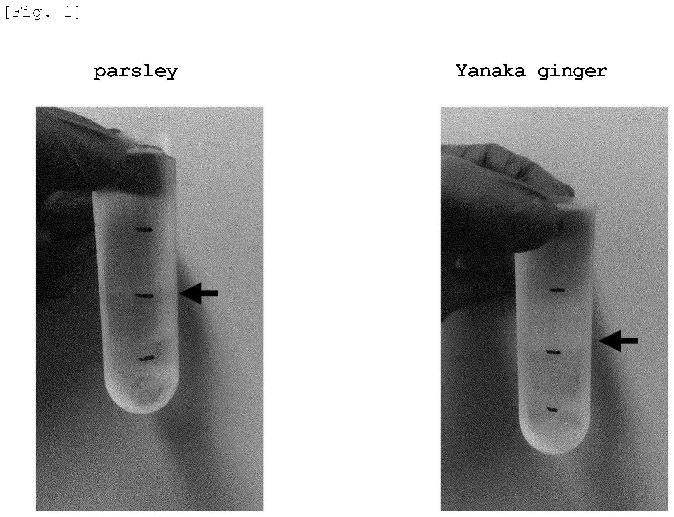

EVs fraction solutions derived from plants and mushrooms described in Table 1 were prepared. First, the plants and mushrooms were washed with water in a plastic bucket, drained, and then made into juice using a household juicer (HUROM). This juice was centrifuged using Avati J-E (JA-14 rotor, manufactured by Beckman Coulter) at 3,000 g for 20 min and further at 10,000 g for 40 min twice, thereby removing larger debris. Then, the obtained supernatant was ultracentrifuged at 150,000 g for 90 min using Optima XE-90 (SW32 Ti rotor, manufactured by Beckman Coulter) to obtain a precipitate. Since EVs were contained in this precipitate, the precipitate was suspended in PBS and then layered onto a sucrose density gradient solution (8, 30, 45, 60%), and further ultracentrifuged at 150,000 g for 90 min. After ultracentrifugation, the Band 2 fraction accumulated at the 30-45% interface was collected using a 20 ml syringe (manufactured by Terumo), mixed with PBS, and then ultracentrifuged at 150,000 g for 90 min, and the precipitate was suspended in PBS to obtain an EVs fraction solution. Fractions of parsley and ginger after layering onto the sucrose density gradient solution and ultracentrifugation are shown in Fig. 1. The arrow indicates the Band 2 fraction. The Band 2 fractions of parsley and ginger had a density of 1.18 g/ml or less.

For microalgae, the culture solution was centrifuged at 3,000 g for 20 min, microalgae were collected, suspended in PBS, freeze-thawed, and then disrupted using an ultrasonic disruption apparatus (manufactured by Branson Sonifier). The disrupted solution was centrifuged at 3,000 g for 20 min and further at 10,000 g for 40 min twice, thereby removing larger debris. Then, the obtained supernatant was ultracentrifuged at 150,000 g for 90 min to obtain a precipitate. Since this precipitate contained EVs, the precipitate was suspended in PBS and used, or layered onto a sucrose density gradient solution (8, 30, 45, 60%), and further ultracentrifuged at 150,000 g for 90 min. After ultracentrifugation, the Band 2 fraction accumulated at the 30-45% interface was collected using a 20 ml syringe (manufactured by Terumo), mixed with PBS, and then ultracentrifuged at 150,000 g for 90 min, and the precipitate was suspended in PBS to obtain an EVs fraction solution.

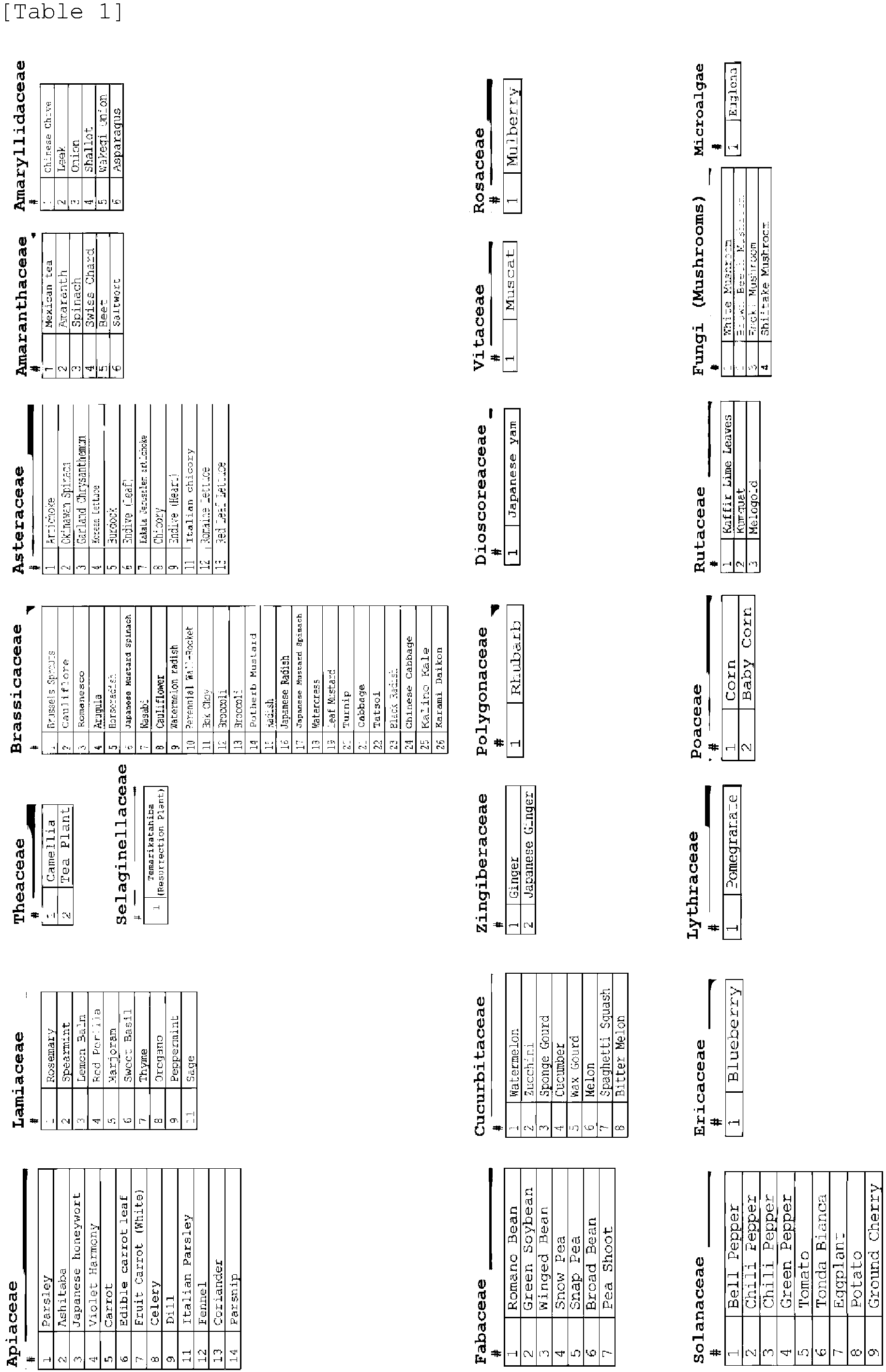

### (1-5) Preparation of EVs samples using ultrafiltration membrane

Winged bean, parsley, and Yanaka ginger were washed with water in a plastic bucket, drained, and then made into juice using a household juicer (HUROM). This juice was centrifuged using Avati J-E (JA-14 rotor, manufactured by Beckman Coulter) at 3,000 g for 20 min and further at 20,000 g for 40 min, thereby removing larger debris. The obtained supernatant was passed through a Supracap depth capsule filter (manufactured by Cytiva), and then subjected to ultrafiltration using a TFF Omega membrane (manufactured by Cytiva) to obtain an EVs sample.

### (1-6) LPS stimulation treatment

An EVs fraction solution or EVs sample prepared using sucrose density gradient ultracentrifugation or an ultrafiltration membrane, having an EVs concentration of 1.0x10¹⁰ particles/ml, was allowed to sensitize mouse macrophage cells seeded in a 12 well dish at 0.8x10⁵ cells/well for 16 hr, followed by washing with PBS, and stimulation with 1 µg/ml LPS (derived from Salmonella minnesota R595, manufactured by WAKO) for 24 hr to induce inflammation. After stimulation, the cells were washed with PBS, and RNA was extracted from the mouse macrophage cells using TRIsure (Nippon Genetics).

### (1-7) Evaluation of effects of EVs on the expression of senescence markers in human IMR90 cells with Ras-induced senescence and on the expression of inflammatory cytokines in mouse macrophage cells after LPS stimulation

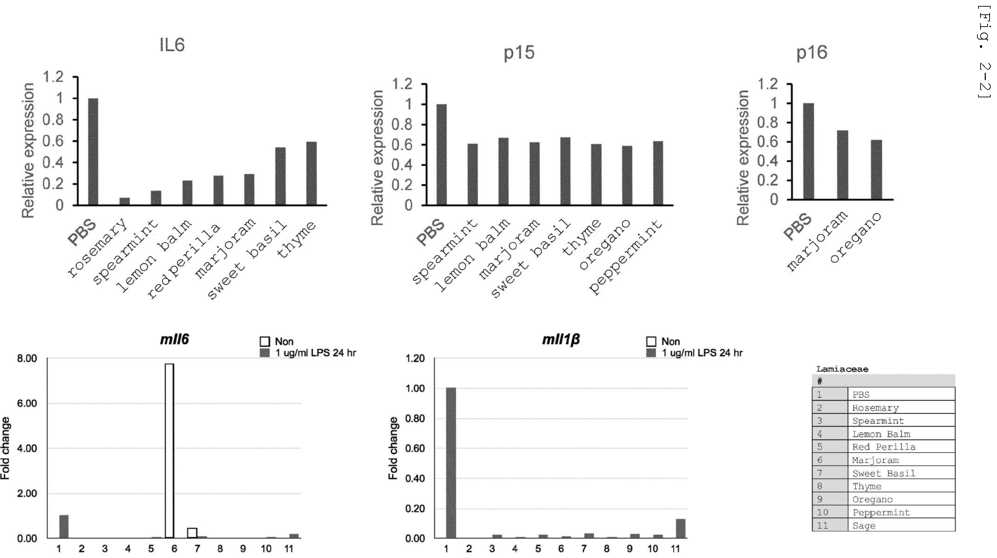

IMR90 cells were seeded in a 12 well dish at 4-6x10⁴ cells/well 24 hr before addition of the EVs fraction solution or EVs sample. An EVs fraction solution or EVs sample prepared by sucrose density gradient ultracentrifugation or ultrafiltration was added to the IMR90 cells at a concentration of 1x10¹⁰ particles/ml. At the same time, activated Ras was overexpressed by adding 4-hydroxytamoxifen to the IMR90 cells, thereby inducing cellular senescence. Thereafter, RNA was extracted from the IMR90 cells, expression levels of cellular senescence markers (p15, p16, Il6) were measured by RT-qPCR, and the aging-suppressing effect of the EVs was evaluated. The results are shown in Fig. 2.

Using expression of inflammatory cytokines (IL-6 and IL-1β) in mouse macrophage cells after LPS stimulation as an indicator, the effect of EVs on the expression of inflammatory cytokines in mouse macrophage cells after LPS stimulation was evaluated. Expression levels of IL-6 and IL-1β were measured by RT-qPCR using RNA extracted from mouse macrophage cells after LPS stimulation.

The results regarding the expression of senescence markers in Ras-induced senescent human IMR90 cells, and the expression of IL-6 and IL-1β in LPS-stimulated mouse macrophage cells, sensitized with sucrose density gradient ultracentrifugation-prepared EVs fraction solutions derived from the plants, mushrooms, or microalgae described in Table 1, or with PBS, are shown in Figs. 2-1 to 2-21.

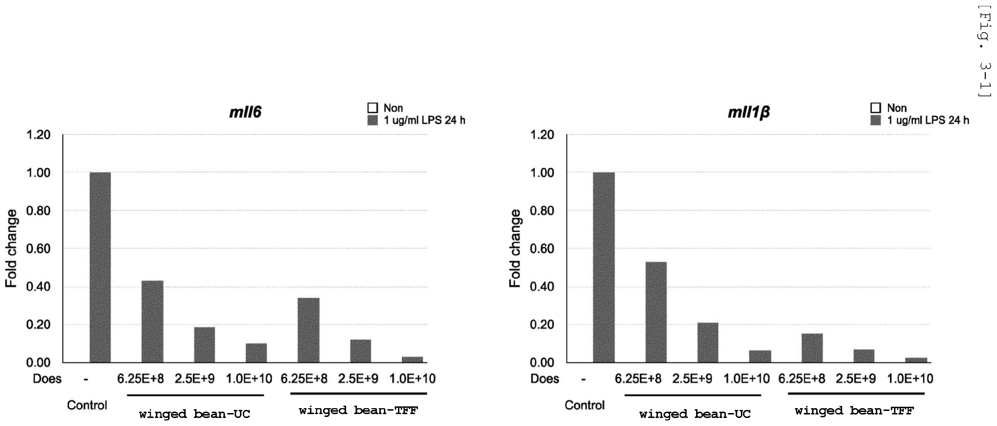

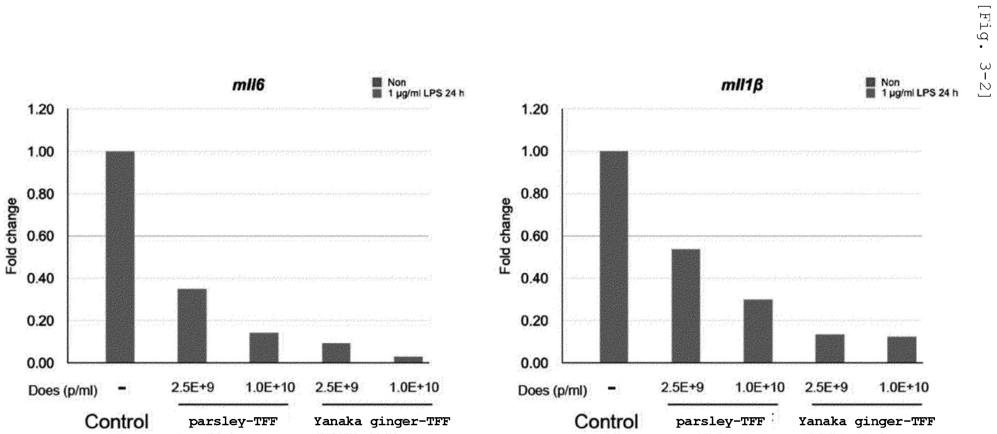

In addition, the results regarding the expression of IL-6 or IL-1β in LPS-stimulated mouse macrophage cells, sensitized with various concentrations of EVs fraction solutions or EVs samples obtained by sucrose density gradient ultracentrifugation or ultrafiltration, or with PBS (Control), are shown in Figs. 3-1 and 3-2. The results of EVs fraction solutions or EVs samples derived from winged bean at each concentration, obtained by sucrose density gradient ultracentrifugation or ultrafiltration, are shown in Fig. 3-1, and the results of EVs samples derived from parsley and Yanaka ginger at each concentration, obtained by ultrafiltration (TFF), are shown in Fig. 3-2.

The expression results (vertical axis) in Figs. 2-1 to 2-21 and Figs. 3-1 and 3-2 are shown as relative values (Fold change), using Gapdh as a normalization gene and taking as 1 the expression level in cells stimulated with Ras or LPS after addition of PBS instead of an EVs fraction solution or EVs sample. The horizontal axis in Fig. 3-2 indicates EVs concentration (particles/mL).

From the results of Figs. 2-1 to 2-21, it became clear that the expression of inflammatory cytokines was markedly reduced in mouse macrophage cells exposed to EVs fraction solutions derived from many of the plants, mushrooms, or microalgae shown in Table 1, as compared with the case where PBS was added.

Further, from the results of Fig. 3-1 and Fig. 3-2, EVs derived from winged bean, parsley, and Yanaka ginger suppressed expression of inflammatory cytokines in mouse macrophage cells in a dose-dependent manner. Regarding the EVs sample derived from winged bean prepared by ultrafiltration, they exhibited a more effective suppressive ability than an equivalent amount of the EVs fraction solution prepared by sucrose density gradient ultracentrifugation.

### (1-8) Nanoparticle Tracking Analysis (NTA: Nano Tracking Analysis)

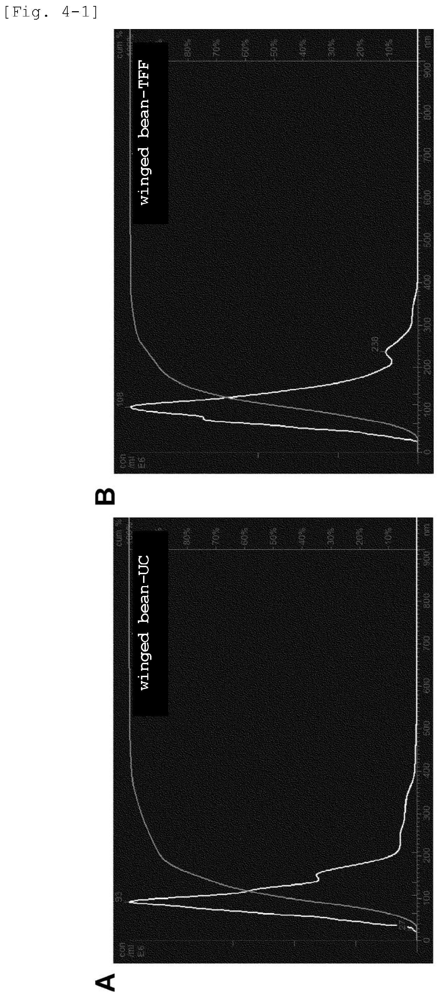

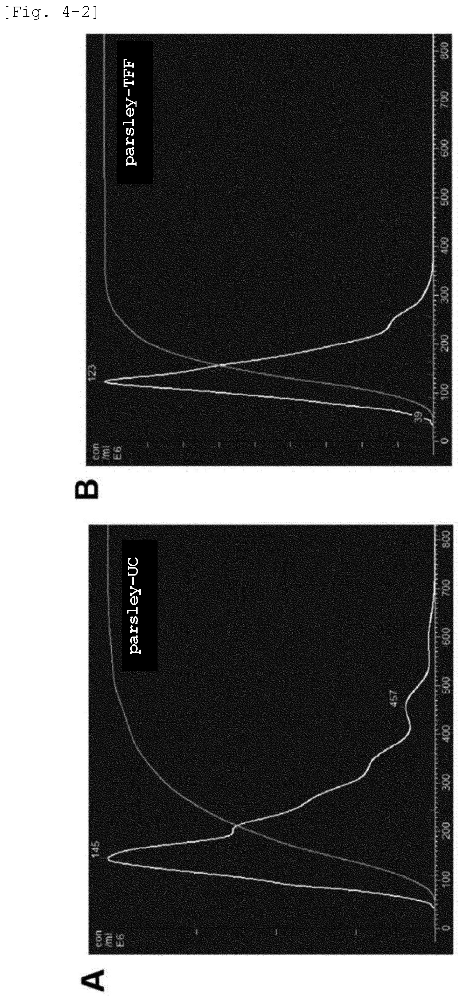

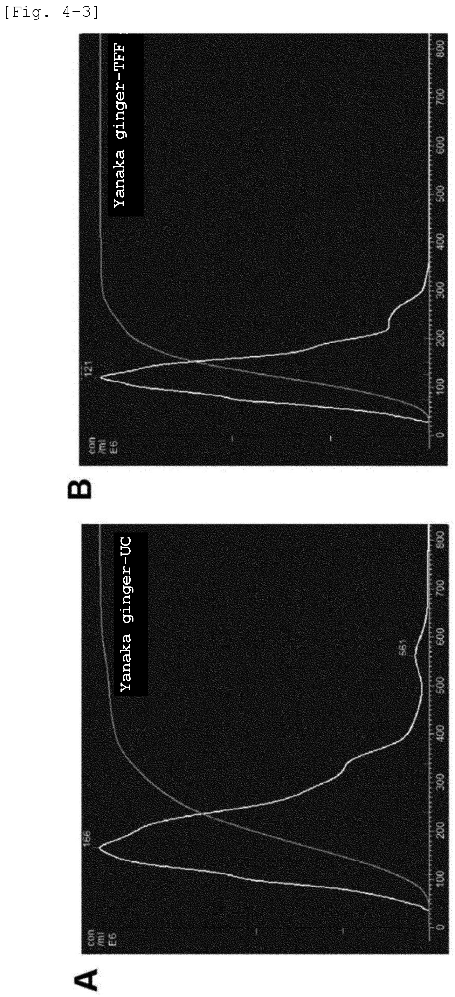

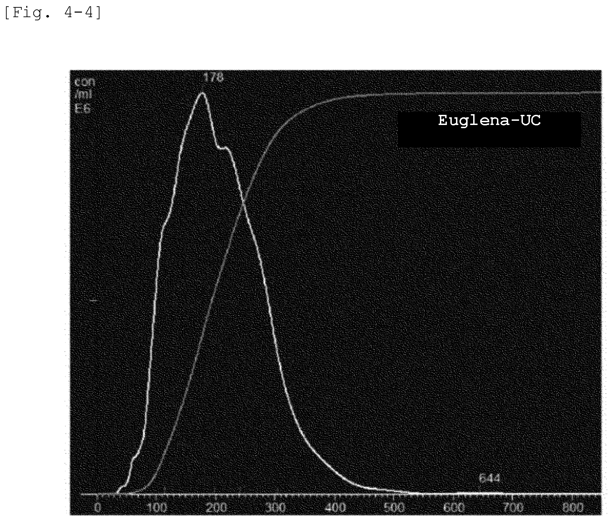

Particle size, particle size distribution, and concentration of EVs in EVs fraction solutions derived from winged bean, parsley, Yanaka ginger, and Euglena, obtained by sucrose density gradient ultracentrifugation, and in EVs samples derived from winged bean, parsley, and Yanaka ginger, obtained by ultrafiltration, were measured using NanoSight NS300 or LM10 (manufactured by Malvern Instruments) equipped with a 405 nm blue laser. The results for winged bean are shown in Fig. 4-1, the results for parsley are shown in Fig. 4-2, the results for Yanaka ginger are shown in Fig. 4-3, and the results for Euglena are shown in Fig. 4-4. Figs. 4-1A, 4-2A, 4-3A, and 4-4 show the results of EVs fraction solutions prepared by sucrose density gradient ultracentrifugation (UC), and Figs. 4-1B, 4-2B, and 4-3B show the results of EVs samples prepared by ultrafiltration (TFF).

From the results of Figs. 4-1 to 4-4, it was found that the EVs fraction solutions derived from winged bean, parsley, Yanaka ginger, and Euglena, prepared by sucrose density gradient ultracentrifugation, contained EVs having sizes of an average of 128 nm (50-500 nm), an average of 216 nm (50-500 nm), an average of 215 nm (50-500 nm), and an average of 211 nm (50-500 nm), respectively. Further, it was found that the EVs samples derived from winged bean, parsley, and Yanaka ginger, prepared by ultrafiltration, contained EVs having sizes of an average of 125 nm (50-300 nm), 148 nm (50-300 nm), and 135 nm (50-300 nm), respectively. From the above, it was found that ultrafiltration can prepare EVs having a more limited size than sucrose density gradient ultracentrifugation.

### <Example 2>

### (2) Effects on improving human skin

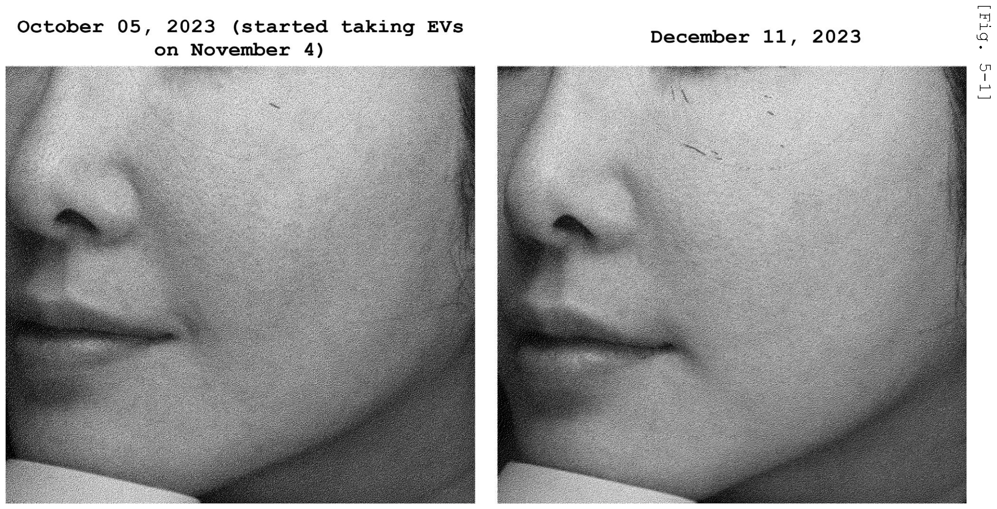

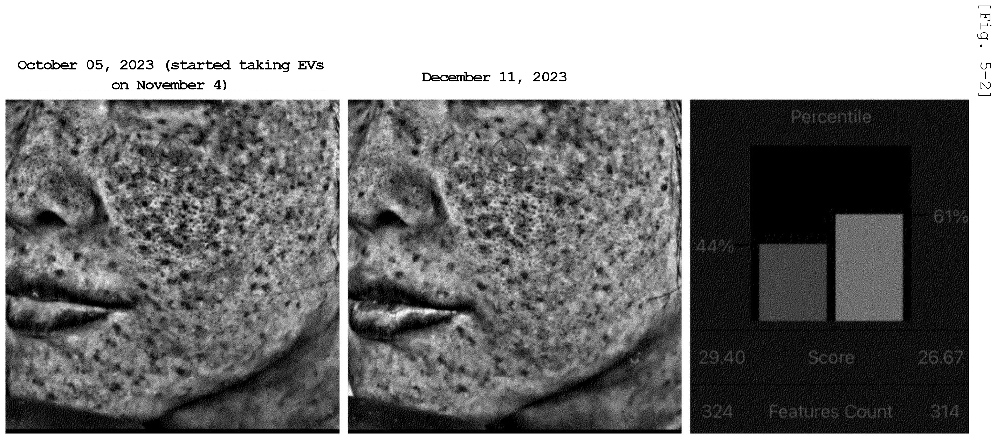

Freeze-dried parsley EVs suspended in water were orally ingested at 8x10¹² particles/day before going to bed every day for 5 weeks. Thereafter, the condition of facial skin was analyzed by a skin diagnostic imaging system VISIA (Figs. 5-1 and 5-2). From the results of Fig. 5-1, it was shown that parsley EVs markedly improve whitening, skin turnover, pores, color unevenness, spots (senile lentigines, melasma, Post-Inflammatory Hyperpigmentation), pigmentation, redness, dullness, texture, firmness, and sagging. Further, from the results of measuring subsurface melanin in Fig. 5-2, melanin, which is the source of dark spots, was metabolized and markedly reduced in size (Fig. 5-2, left diagram and middle diagram), and the percentile value, which indicates the population percentage from the bottom when compared with individuals of the same age who underwent VISIA analysis, increased (Fig. 5-2, right diagram), thus showing that parsley EVs markedly improve latent spots (invisible dark spots). Therefore, parsley EVs are considered to have a skin-improving effect.

### <Example 3>

### (3) Stability of heat-treated EVs

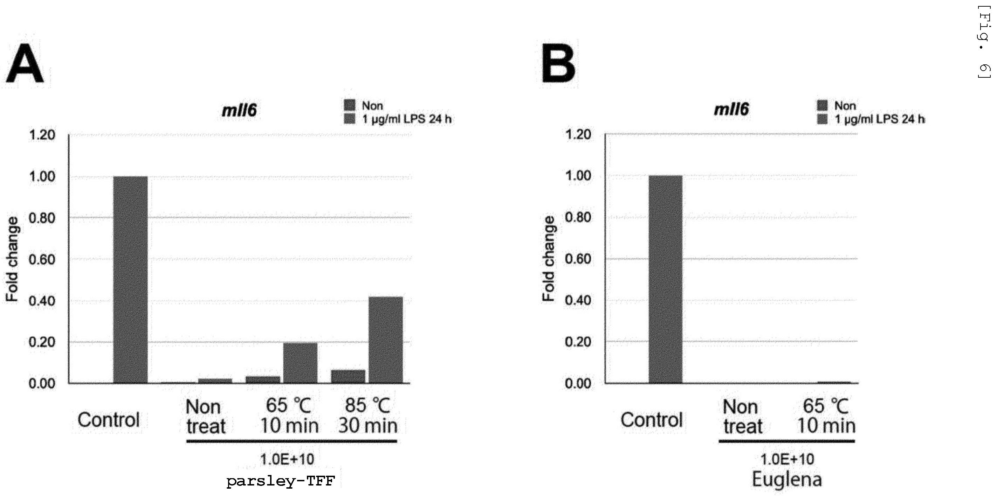

After treating the prepared EVs solution under heat treatment conditions used commonly for food products (65°C for 10 min or 85°C for 30 min), it was added to the medium to 1x10¹⁰ particles/mL to sensitize mouse macrophage cells (Raw-Blue cells) for 16 hr. Subsequently, they were stimulated with 1 µg/mL LPS for 24 hr, and washed with PBS. Thereafter, RNA was extracted using TRIsure, cDNA was synthesized by a reverse transcription reaction, and then expression of IL-6 was confirmed by qPCR. As a control, cells in which an equal amount of PBS was added into the medium instead of the EVs solution were used, and a relative value where the expression level of IL-6 in the control cells was taken as 1 was shown (Fig. 6).

From the results of Fig. 6-A, the plant-derived (parsley-derived) EVs, even when subjected to heat treatment at 65°C or 85°C, did not show any attenuation in their efficacy compared to unheated EVs. From the results of Fig. 6-B, the microalgae-derived (Euglena-derived) EVs also, even when subjected to heat treatment, did not show any attenuation in their efficacy compared to non-heat-treated EVs. Therefore, it was considered that parsley- and microalgae-derived EVs have heat resistance ability. Accordingly, parsley- and microalgae-derived EVs can be simply sterilized and made into products without losing activity due to heat treatment.

### <Example 4>

### (4) Anti-inflammatory effect in mouse colitis model mice

After wild-type, 8-week-old male mice (C57BL/6J) were administered 1x10¹¹ parsley-derived EVs once a day for 1 week, 3% dextran sulfate sodium (DSS) was added to their drinking water to induce colitis, and the same dose of parsley-derived EVs was continuously administered.

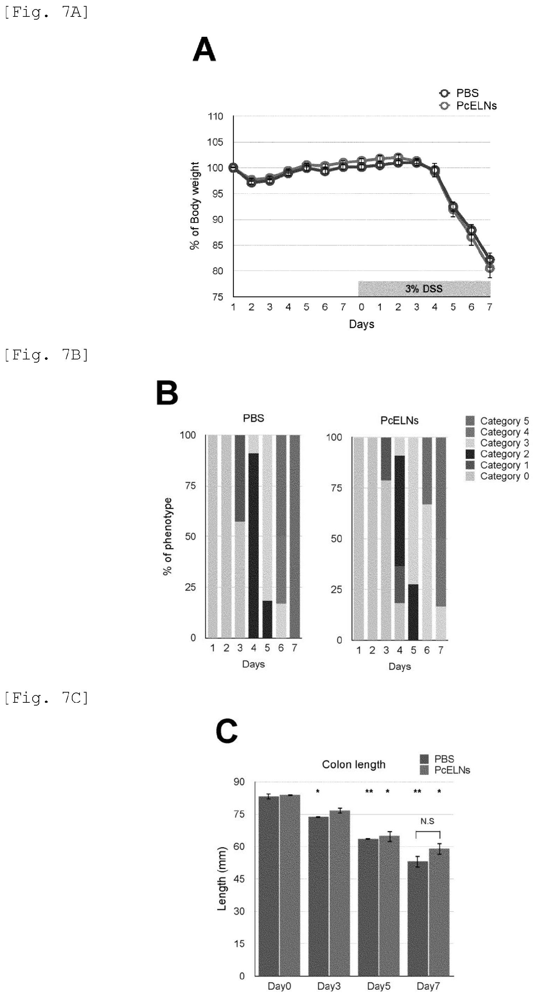

As shown in Fig. 7A, at the stage of no DSS treatment, body weight increase similar to that in the PBS ingestion group used as a control was observed in the mouse group to which parsley-derived EVs were administered, and acute toxicity to individuals by parsley-derived EVs was not recognized.

As shown in Fig. 7B, from day 3 of DSS ingestion, contamination of blood in feces was observed in the PBS ingestion group and the parsley-derived EVs ingestion group, and increased according to the number of administration days. Bleeding from the anus was observed in 50% of mice on day 6 and in 100% of mice on day 7 in the PBS ingestion group, whereas in the parsley-derived EVs ingestion group, it was observed only in 30% of mice on day 6 and in 50% of mice even on day 7. As shown in Fig. 7C, the length of the colon from the cecum to the anus significantly decreased from day 3 onward in the PBS ingestion group, but in the parsley-derived EVs ingestion group, a significant difference was observed from day 5 onward.

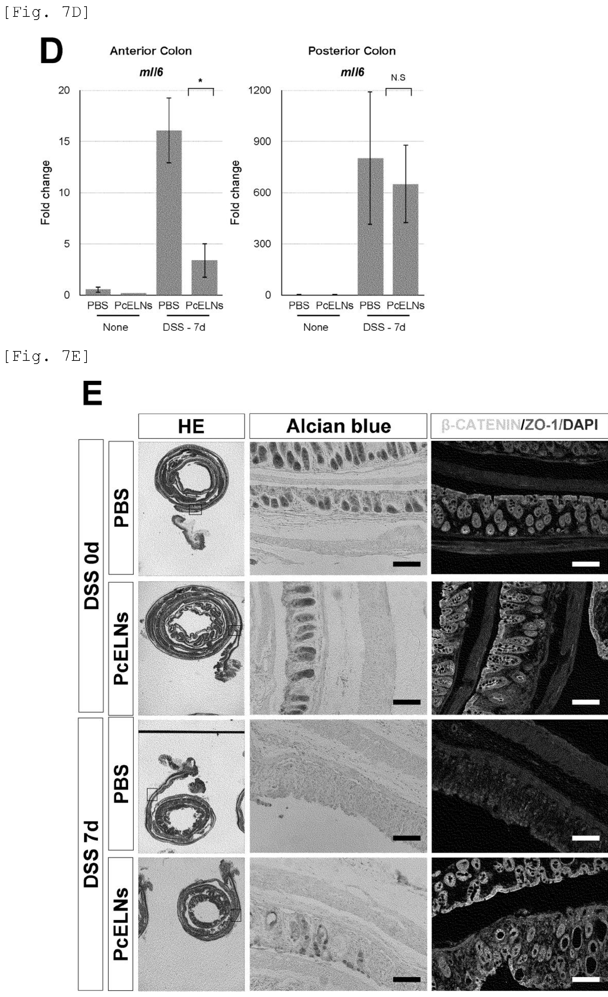

Then, the intestine was divided from the region close to the cecum into the anterior, middle, and posterior colon regions, and after RNA extraction, expression fluctuations of the inflammatory cytokine Il6 were verified by qPCR. As shown in Fig. 7D, in the parsley-derived EVs ingestion group, the expression on day 7 of DSS ingestion was significantly suppressed compared to that in the PBS ingestion group.

Further, after DSS was ingested, perfusion fixation was performed on days 0 and 7, paraffin sections were prepared by making a Swiss roll of the colon region from the cecum to the anus, HE staining and Alcian blue staining (pH 2.5) were performed to observe the morphology of the colon sections. As shown in Fig. 7E, on day 0 of DSS ingestion, in both the PBS ingestion group and the parsley-derived EVs ingestion group, the staining area of Alcian blue, which binds to the carboxyl groups and sulfate groups of acidic mucopolysaccharides, was observed over almost the entire region including the posterior colon region (the region enclosed by a square in the left diagram of Fig. 7E). In contrast, in the PBS ingestion group on day 7 of DSS ingestion, the Alcian blue staining area almost disappeared in the posterior colon region, whereas in the parsley-derived EVs ingestion group, the Alcian blue staining area was present also in the posterior colon region. Furthermore, also in immunostaining images using β-catenin and ZO-1, which are epithelial cell markers, in the PBS ingestion group on day 7 of DSS ingestion, a remarkable defect of the simple columnar epithelial structure was observed, whereas in the parsley-derived EVs ingestion group, the epithelial structure was maintained.

### <Example 5>

### (5) Dermatitis treatment effect

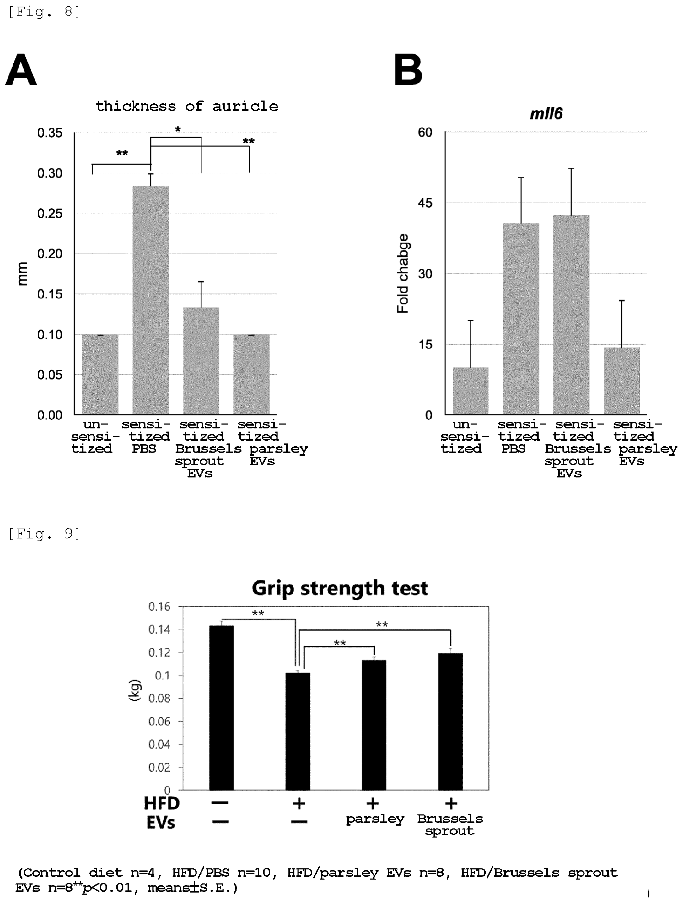

The abdomens of 8-week-old C57BL/6J male mice were shaved, and 150 µl/mouse of 5% TNCB (2,4,6-trinitrochlorobenzene: Tokyo Chemical Industry) dissolved in an acetone:ethanol (1:9) solution was applied thereto to perform sensitization. Six days later, inflammation was induced by applying 10 µl each of 1% TNCB dissolved in acetone to the front and back surfaces of both ears. Acetone was applied to the non-inflammation-induced group. Six hours after the induction of inflammation, PBS or a solution containing 1.8x10¹⁰ particles/µl Brussels sprout-derived EVs or parsley-derived EVs was applied dropwise in an amount of 10 µl each onto the front and back surfaces of both ears, and was rubbed with a finger to allow penetration. The results obtained by measuring the thickness of the auricle with a micrometer 18 hr later are shown in Fig. 8A. Measurement was performed for both ears, and the average value thereof was taken as the auricle thickness of the individual. From the results of Fig. 8A, it was found that ear swelling accompanying inflammation was significantly suppressed in the group administered Brussels sprout-derived EVs or parsley-derived EVs as compared with the PBS group as a control.

Then, after measuring the ear thickness, RNA was collected from the auricle of the left ear, and the expression levels of inflammatory cytokines were measured by RT-qPCR. The results are shown in Fig. 8B.

### <Example 6>

### (6) Evaluation of influence of plant EVs on obese mice

In order to quantitatively evaluate the influence of parsley- and Brussels sprout-derived EVs on aging and muscle loss associated with obesity in individual mice, mice were fed a high-fat diet for 13 weeks to induce obesity, and during the same period, parsley- and Brussels sprout-derived EVs were administered via drinking water to the mice. A Grip Strength Test for measuring muscle strength (grip strength) and a 4-limb Hanging Test were performed on a non-obese mouse group (HFD-), an obese mouse group (HFD+), and groups in which obese mice were given parsley-derived EVs or Brussels sprout-derived EVs, respectively. In addition, the soleus muscle weight of each mouse was measured.

Specifically, 5-week-old C57BL/6J mice were obtained from Charles River, acclimated for 1 week, and then divided into three groups such that the average body weight of each group was uniform. Each mouse was fed a high-fat diet for 13 weeks, and simultaneously administered pure water containing parsley- or Brussels sprout-derived EVs at a concentration of 3×10⁹ particles/mL. D12450B (Research Diet) was freely fed as a normal diet, and D12492 (Research Diet) was freely fed as a high-fat diet. The high-fat diet and pure water containing each EV were replaced with new ones twice a week.

The Grip Strength Test was performed on the obese mice administered each EV using a smart-type grip strength meter for rats and mice, MK-380Si (manufactured by Muromachi Kikai). Three measurements were conducted per mouse, and the average value thereof was taken as the grip strength value of each mouse. The results are shown in Fig. 9.

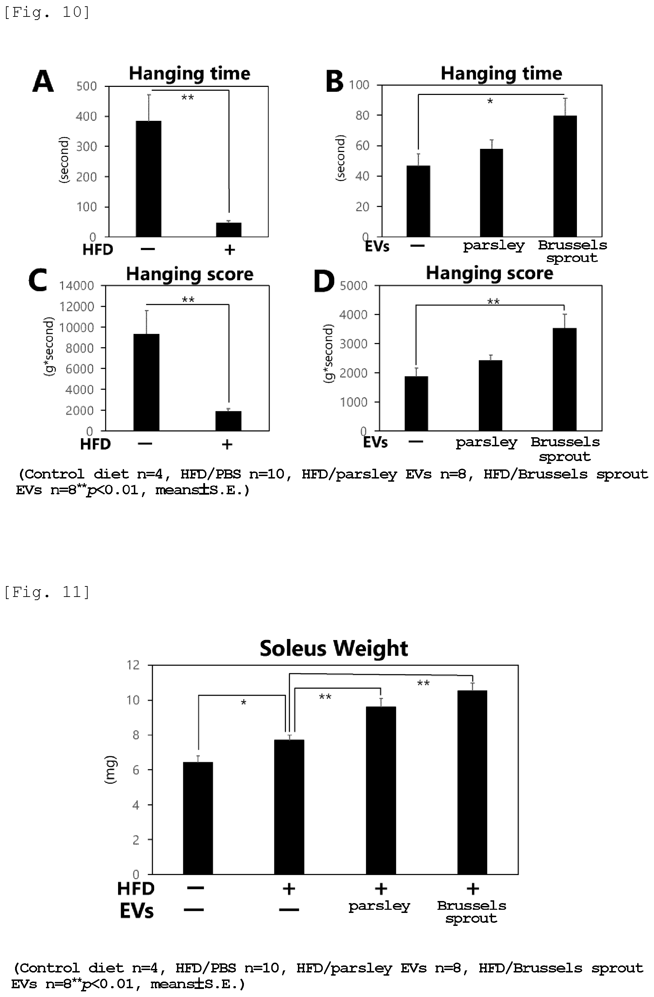

The 4-limb Hanging Test was performed using a wire hanging test box (manufactured by O'HARA & Co., Ltd.), and under conditions where the mouse was gripping the wire mesh with all four limbs, the wire mesh was inverted upside down together with the mouse. The duration for which the mouse endured before falling was defined as the Hanging time, and the value obtained by multiplying this endurance time by the body weight of each mouse was defined as the Hanging score. The results are shown in Fig. 10.

The soleus muscle was collected from the hind limbs of each mouse administered the high-fat diet and each EV for 13 weeks, and the weight thereof was measured and evaluated. The results are shown in Fig. 11.

In the Grip strength test, a decrease in grip strength was observed in the high-fat diet-fed group due to obesity associated with the high-fat diet load, however, the mouse groups administered parsley-derived EVs or Brussels sprout-derived EVs both showed higher grip strength values compared with the control obese mouse group (Fig. 9). In the 4-limb Hanging Test, decreases in the endurance time in the upside-down hanging state (Hanging time) (Fig. 10-A) and the score obtained by multiplying the endurance time by mouse body weight (Hanging score) (Fig. 10-C) were observed due to obesity. However, in the obese mouse group administered Brussels sprout-derived EVs, both the Hanging time (Fig. 10-B) and the Hanging score (Fig. 10-D) were significantly greater compared to those of the control obese mouse group, indicating that the obese mice administered Brussels sprout-derived EVs have high muscle endurance. Although no significant difference was observed in the obese mouse group administered parsley-derived EVs in the 4-limb Hanging Test, the group showed a tendency that reduction in muscle endurance was suppressed (Figs. 10-B, D).

Furthermore, when the soleus muscle was isolated from the hind limbs of each mouse and the weight thereof was measured, it was clarified that the soleus muscle weight was significantly increased in the obese mouse groups administered parsley-derived EVs and Brussels sprout-derived EVs as compared with the control obese mouse group (Fig. 11).

As described above, parsley-derived EVs and Brussels sprout-derived EVs were considered to have an effect of suppressing reduction in muscle strength and muscle endurance, namely, an aging-suppressing effect.

## Claims

1. An anti-inflammatory or aging-suppressing composition, comprising an extracellular vesicle derived from a plant of the family Apiaceae, the family Lamiaceae, the family Theaceae, the family Selaginellaceae, the family Brassicaceae, the family Asteraceae , the family Amaranthaceae, the family Amaryllidaceae, the family Fabaceae, the family Cucurbitaceae, the family Zingiberaceae, the family Polygonaceae, the family Dioscoreaceae, the family Vitaceae, the family Rosaceae, the family Solanaceae, the family Ericaceae, the family Lythraceae, or the family Rutaceae, a mushroom, or a microalga.

2. An anti-inflammatory or aging-suppressing composition, comprising an extracellular vesicle derived from parsley, ashitaba, Japanese honeywort, Violet Harmony, carrot, edible carrot leaf, fruit carrot, coriander, dill, fennel, rosemary, spearmint, lemon balm, perilla, marjoram, sweet basil, thyme, oregano, peppermint, sage, tea plant, camellia, Temarikatahiba, Brussels sprouts, cauliflore, romanesco, arugula, horseradish, Japanese mustard spinach, wasabi, cauliflower, watermelon radish, perennial wall-rocket, bok choy, broccoli, potherb mustard, radish, Japanese radish, watercress, turnip, tatsoi, artichoke, Okinawan spinach, garland chrysanthemum, Korean lettuce, burdock, endive, Hakata Jerusalem artichoke, romaine lettuce, Italian chicory, Mexican tea, amaranthus, spinach, Swiss chard, beet, saltwort, Chinese chive, leek, onion, shallot, Romano bean, green soybean, winged bean, snow pea, snap pea, pea shoot, watermelon, zucchini, sponge gourd, ginger, Japanese ginger, rhubarb, Japanese yam, muscat, mulberry, bell pepper, chili pepper, potato, green pepper, blueberry, pomegranate, kaffir lime, kumquat, white mushroom, shiitake mushroom, brown beech mushroom, or Euglena.

3. The composition according to claim 1 or 2, wherein the composition suppresses expression of an inflammatory cytokine or an aging marker.

4. The composition according to claim 3, wherein the inflammatory cytokine or aging marker is IL-4, IL-6, IL-13, IL-33, IL-1β, CXCL1, CXCL2, TNF-α, p15, or p16.

5. The composition according to claim 1 or 2, for a food, a cosmetic, or a pharmaceutical product.

6. An anti-inflammatory or aging-suppressing composition, comprising an extracellular vesicle derived from parsley or Euglena.

7. The composition according to claim 6, wherein an anti-inflammatory or aging-suppressing action is not attenuated when the composition is subjected to a thermal history.

8. The composition according to claim 7, wherein the thermal history is heat treatment performed at 40°C or higher.
